# EUROPEAN PATENT SPECIFICATION

(11) **EP 2 150 246 B1**
(45) Date of publication and mention of the grant of the patent: **25.09.2013**
(21) Application number: 08742891.8
(22) Date of filing: 14.04.2008
(51) Int. Cl.: A61K 31/198, A61K 31/205, A61K 31/519, A61P 25/24

(54) **COMPOSITIONS AND METHODS FOR TREATMENT OF PSYCHIATRIC DISORDERS**
ZUSAMMENSETZUNGEN UND VERFAHREN ZUR BEHANDLUNG VON PSYCHIATRISCHEN ERKRANKUNGEN
COMPOSITIONS ET PROCÉDÉS DE TRAITEMENT DE TROUBLES PSYCHIATRIQUES

(30) Priority: 13.04.2007 US 923310 P
(43) Date of publication of application: 10.02.2010
(73) Proprietor: Dunn, Robert T., Yonkers NY 10710 (US)
(72) Inventor: DUNN, Robert, Bedford, MA 01730 (US)
(74) Representative: Mintz Levin Cohn Ferris Glovsky and Popeo LLP
(86) International application number: PCT/US2008/004835
(87) International publication number: WO 2008/127725

(56) References cited:
- WO-A-00/25764
- WO-A-96/33703
- US-A- 5 597 585
- PROCTER A: "Enhancement of recovery from psychiatric illness by methylfolate." THE BRITISH JOURNAL OF PSYCHIATRY : THE JOURNAL OF MENTAL SCIENCE AUG 1991, vol. 159, August 1991 (1991-08), pages 271-272, XP009101735 ISSN: 0007-1250
- GUARALDI G P ET AL: "AN OPEN TRIAL OF METHYLTETRAHYDROFOLATE IN ELDERLY DEPRESSED PATIENTS" ANNALS OF CLINICAL PSYCHIATRY, ELSEVIER, US, vol. 5, no. 2, 1 January 1993 (1993-01-01), pages 101-105, XP000980561 ISSN: 1040-1237

## Description

### FIELD OF THE TECHNOLOGY

This invention relates to clinical depression and treatment thereof.

### BACKGROUND

Depression is a prevalent illness which affects the lives of many people each year. Antidepressant drugs are the mainstay of treatment for depression. Depressed patients, however, often fail to take needed medication reliably. At least 10% of depressed patients do not benefit from available treatments, and many are intolerant to adverse side effects, including sexual dysfunction and weight-gain. There remains a need for effective and tolerable antidepressant treatments.

### SUMMARY

This invention provides a therapeutic formulation that includes a mixture of betaine, L-methionine and folate or folic acid in an amount that augments the endogenous synthesis of S-adenosylmethionine (SAM) produced in the one-carbon cycle for use in the treatment of unipolar depression. Folate, as defined in this invention, is the anion form of folic acid. In one embodiment, the composition consists essentially of betaine, folate or folic acid, L-methionine and a pharmaceutically acceptable excipient Preferably, the compositions do not include cobalamin.

Patients treated are identified as suffering from or being at risk of developing unipolar depression. Preferably, the subjects are not characterized or diagnosed as having bipolar depression. Symptoms of unipolar depression typically include sleeping and/or eating less (typical depression); however, the symptoms may manifest as sleeping and/or eating more (atypical depression). The compositions are effective in reducing the symptoms of typical depression as well as atypical depression characterized by increased sleep and increased appetite. The therapeutic formulation that is administered maintains homocysteine levels in the patient within a normal range (e.g., 5-15 µ moles per liter).

Alternatively, the formulation does not substantially increase the homocysteine level in an individual (e.g., if the homocysteine level of an individual is in the moderate (16-20 µmoles per liter), intermediate (31-100 µmoles per liter) or severe (>100 µmoles per liter) range, the level remains in the same range or increases by less than 30%). Preferably, the increase is less than 20%. For example, an increase in homocysteine level does not exceed 10 µmoles per liter compared to a pre-treatment level. Preferably, the increase is less than 5 µmoles per liter compared to the level prior to commencement of treatment. The therapeutic formulation produces SAM in an amount that is between 100-180%, 120-160%, or 140% times the average endogenous amount of SAM produced in the person prior to administration.

The compounds are for administration in an effective amount to alleviate one or more symptoms of unipolar depression. The effective amount of active compound(s) varies depending upon the route of administration, age, body weight, and general health of the subject. A physician or other medical profession determines the appropriate amount and dosage regimen for an individual subject using standard methods..

The dosage of the therapeutic formulation includes between 1-6, 1.5-4, or 2.5 times the daily requirement of L-methionine of the patient. Furthermore, the formulation includes between 1-20, 2.5-15, or 5-10 times the average daily intake of betaine of the patient. Moreover, the formulation includes 1-20, 10-18, or 13 times the daily requirement of folate of the patient. In other words, the dosage is in the range of 1-10, 3-8, or 5 g/day of L-methionine. The dosage also includes between 1-20, 8-15, or 10 g/day of betaine. The dosage also includes between 1-15, 3-10, or 5 meg/day of folate. The dosage overall comprises a combination of 5 g/day of L-methionine, 10 g/day of betaine and 5 mcg/day of folate. The dosage is administered for at least 4 or at least 6 weeks. The compounds L-methionine, betaine, and folate or folic acid are administered individually or are combined and added to a base substance. Exemplary base or vehicles include a bar (e.g., a protein or energy bar), a liquid, a cookie, and a powder. In the latter case, the powder is optionally mixed with a liquid such as water, a fruit juice, or dairy product, e.g., milk or yogurt, to yield a shake or smoothie.

The compositions and methods provide numerous advantages compared to traditional drugs used for treatment of depression. For example, clinical efficacy, i.e., alleviation of symptoms of unipolar depression, coupled with fewer and more tolerable side effects lead to greater patient compliance. Thus, both typical and atypical depressive symptoms are improved by the L-methionine, betaine, and folate or folic acid treatment, without the side effects observed with antidepressant drugs, such as weight gain, sexual dysfunction, excess sedation, insomnia, hypertension, orthostatic hypotension, abnormal liver function tests, or electrocardiographic changes. The L-methionine, betaine, and folate or folic acid combination treatment exerts its effects more quickly than antidepressant drugs, with significant benefits observed at day 3 or 4 of treatment, whereas antidepressant drugs take 2-6 weeks to be effective.

Other features and advantages of the invention will be apparent from the following description of the preferred embodiments thereof, and from the claims.

### BRIEF DESCRIPTION OF THE FIGURES

Figure 1 is a line graph showing the Hamilton Depression Rating Scale average scores versus the week.
Figure 2 is bar graph showing the comparison of Clinical Global Impression - severity, Beck Depression Inventory at baseline and endpoint.
Figure 3 is a bar graph showing the comparison of Zung Anxiety Rating Scale and Brief Psychiatric Rating Scale at baseline and endpoint.
Figure 4 is bar graph showing a comparison of Young Mania Rating Scale and Psychiotic Symptom Rating Scale at baseline and endpoint.
Figure 5 is a bar graph showing the Hamilton Depression Rating Scale Score decreased from baseline to endpoint.
Figure 6 is a bar graph showing the Beck Depression Inventory Scores decreased from baseline to endpoint.
Figure 7 is a bar graph showing the Clinical Global Impression-Severity Scale improvement from baseline to endpoint.
Figure 8 is a line graph showing the improves Hamilton Depression Rating Scale scores.
Figure 9 is a bar graph showing the Zung Anxiety Scale Improvement from baseline to endpoint.
Figure 10 is a bar graph showing the Young Mania Rating Scale improvement from baseline to endpoint.
Figure 11 is a bar graph showing the psychosis subscore of the Brief Psychiatric Rating Scale.
Figure 12 is a bar graph showing the Adverse Effects Scale improvement from baseline to endpoint.
Figure 13 shows the NARSAD Evaluation Schedule 1
Figure 14 is a line graph of the Hamilton Depression Rating Scale (28 item version) Score Decrease during Treatment
Figure 15 is a bar graph showing the laboratory values.
Figure 16 is a bar graph of the homocysteine levels of the patients.

### DETAILED DESCRIPTION

Depression is a common illness that produces much human and is characterized by suffering high morbidity and mortality. It affects 1 in 10 people, leads to suicide in 10-20% of its sufferers, increases death rated 4-fold over age 55, and it is the fifth most expensive medical illness in the U.S. Antidepressants drugs are the mainstay of treatment for depression. However, many depressed patients do not take needed medication to treat their depression, and about 10% of depressed patients are refractory to existing antidepressant therapies. Additionally, the adverse effects of antidepressant drugs are intolerable to many patients, preventing effective treatment for their depression. Thus, sexual dysfunction, weight gain, excess sedation, worsened anxiety or insomnia, hypertension, orthostatic hypotension, elevated LFTs and EKG changes often lead to lack of compliance and discontinuation of antidepressant drugs.

Clinical depression, or unipolar depression when compared to bipolar depression, is a state of intense sadness, melancholia or despair that has advanced to the point of being disruptive to an individual's social functioning and/or activities of daily living. Clinical depression differs from depression in that it involves a clinical diagnosis and is acknowledged to be more serious that a person with normal depressed feelings. Clinical depression often leads to constant negative thinking and, on occasion, substance abuse.

People identify being depressed as "feeling sad for no reason" or "having no motivation to do anything." In order to be diagnosed with clinical depression, a person's depressed mood or anhedonia must be present for at least two weeks and be accompanied by other symptoms including feeling tired, sad, irritable, lazy, unmotivated, and apathetic. Other symptoms include an overwhelming fear or inability to feel emotion (emptiness), a decreased amount of pleasure in all or almost all daily activities, a change in weight either loss or gain, disturbed sleep patterns including insomnia, loss of REM sleep, or excessive sleep. Clinically depressed patients can also have a psychomotor agitation or retardation everyday, fatigue, mental or physical, trouble concentration, among others.

Major clinical depression is a unipolar depression. Unipolar depression is when a person is diagnosed with clinical depression and has never been diagnosed symptoms of bipolar mania. Bipolar depression is where a person who has episodes of clinical depression but the person also experiences episodes of mania or a markedly elevated mood.

Treatment of depression varies broadly and differs from one individual to another. Various types and combinations of treatments may have to be tried, before reaching a successful solution to the problem. One mode of treatment is with medication. Alternative treatments used for depression include exercise and the use of vitamins, herbs, or other nutritional supplements. Medication that relieves the symptoms of depression has been available for several decades. Typical first-line therapy for depression is the use of an selective serotonin reuptake inhibitor, such as citalopram (Celexa), fluoxetine (Prozac), paroxetine (Paxil), and sertraline (Zoloft).

Monoamine oxidase inhibitors (MAOIs) such as Nardil may be used if other antidepressant medications are ineffective and there are potentially fatal interactions between this class of medication and certain foods and drugs. MAOIs are used to block the enzyme monoamine oxidase which breaks down neurotransmitters such as serotonin and norepinephrine (noradrenaline). Tricyclic antidepressants are the oldest and include such medications as amitriptyline and desipramine. Tricyclics block the reuptake of certain neurotransmitters such as norepinephrine (noradrenaline) and serotonin, side effects include weight gain, sexual dysfunction, increased heart rate, drowsiness, dry mouth, constipation, urinary retention, blurred vision, dizziness, and confusion. Most importantly, they have a high potential to be lethal in moderate overdose. However, tricyclic antidepressants are still used because of their high potency, especially in severe cases of clinical depression.

Selective serotonin reuptake inhibitors (SSRIs) are a family of antidepressant considered to be the current standard of drug treatment. It is thought that one cause of depression is an inadequate amount of serotonin, a chemical used in the brain to transmit signals between neurons. SSRIs are said to work by preventing the reabsorption of serotonin by the nerve cell, thus maintaining the levels the brain needs to function effectively. This family of drugs includes fluoxetine (Prozac), paroxetine (Paxil), escitalopram (Lexapro), citalopram (Celexa), and sertraline (Zoloft). These antidepressants typically have fewer adverse side effects than the tricyclics or the MAOIs, although such effects as weight gain, sexual dysfunction, loose stools, drowsiness, dry mouth, nervousness, anxiety, insomnia, or decreased appetite. Some side effects may decrease as a person adjusts to the drug, but other side effects may be persistent.

Other medications include Norepinephrine (noradrenaline) reuptake inhibitors (NRIs) such as reboxetine (Edronax) act via norepinephrine (also known as *noradrenaline*), Norepinephrine-dopamine reuptake inhibitors such as bupropion (Wellbutrin, Zyban) inhibit the neuronal reuptake of dopamine and norepinephrine (noradrenaline), and Serotonin- norepinephrine reuptake inhibitors (SNRIs) such as venlafaxine (Effexor) and duloxetine (Cymbalta) are a newer form of antidepressant that works on both noradrenaline and serotonin. They typically have similar side effects to the SSRIs, although there may be a withdrawal syndrome on discontinuation that may necessitate dosage tapering.

Rather than taking a medication, some reports indicate patients can also take dietary supplements that include 5 -HTP supplements, which claim to provide more raw material to the body's natural serotonin production process. Omega-3 fatty acids (found naturally in oily fish, flax seeds, hemp seeds, walnuts, and canola oil) have been found in some studies but not others to be effective when used as dietary supplements. St John's Wort [Hypericum perforatum] can assist with the feelings of melancholia and anxiety, and it is a mood-enhancing herbal substance which acts like an antidepressant and increases the availability of serotonin, norepinephrine and dopamine at the neuron synapses; its primary benefit appears to be in mild-to-moderate depression. Ginkgo Biloba can also be an effective natural antidepressant as it stabifizes cell membranes, inhibits lipid breakdown and aids in cell use of oxygen and glucose. Zinc and Biotin and Vitamin B-12 have also been used as symptoms of a vitamin B- 12 deficiency can include depression and other psychiatric disorders. The amino acids phenylalanine and tyrosine may produce a favorable effect on certain forms of depression. They enhance the neurotransmitters dopamine and noradrenalin. Proctor ("Enhancement of recovery from psychiatric illness by methylfolate", British Journal of Psychiatry: the Journal of Mental Science, vol 159 pp 271-272 (1991)) and Guaraldi et al ("An open trial of methyltetrahydrofolate in elderly depressed patients", Annals of Clinical Psychiatry, vol 5 (2), pp 101-105)) disclose use of 5-methylfolate, alone, in the treatment of depression.

US 5,597,585 relates to vitamin compositions for use as a general dietary supplement and discloses compositions comprising, amongst other components, folic acid, betaine and methionine.

Another dietary supplement includes S-adenosyl methionine (SAM-e), which is a derivative of the amino acid methionine. It acts as a methyl donor and participates in other biochemical reactions. It is available as a prescription antidepressant in Europe and an over-the- counter dietary supplement in the United States. Clinical trials have shown SAM-e to be as effective as standard antidepressant medication, with fewer side effects; however, some studies have reported an increased incidence of mania resulting from SAM-e use compared to other antidepressants. Note that the vast majority of studies finding SAMe effective in depression administered it intravenously or intramuscularly.

Disturbances in the one-carbon cycle have been implicated in affective disorders and agents augmenting the one-carbon cycle have antidepressant effects. The one-carbon cycle involves the generation of SAM, the utilization of the labile methyl group of SAM in transmethylation reactions, and the regeneration of SAM. SAM has been found effective in the treatment of depression, largely in studies utilizing parenteral administration. Described herein, as an alternative to administering exogenous SAM for the treatment of depression, is augmentation of its endogenous synthesis. A regimen of L-methionine, betaine and folate or folic acid augments SAM levels better than exogenous SAM and does not require parenteral administration.

### The One-Carbon Cycle

The one-carbon cycle involves the generation of S-adenosylmethionine (SAM or SAMe) from methionine and ATP by methionine adenosyltransferase (MAT), the utilization of the labile methyl group of SAM in transmethylation reactions, yielding S-adenosylhomocysteine, which is hydrolyzed to homocysteine and in turn methylated to regenerate methionine. Methyl groups generally enter the cycle in the conversion of homocysteine to methionine, catalyzed by homocysteine methyltransferase (aka methionine synthase) and vitamin B 12, with the methyl group supplied by 5-methyltetrahydrofolate (Stryer Biochemistry, WH Freeman, San Francisco, 1981, Reynolds et al., Psychological Medicine, 13:705-710,1983). L-methionine supplementation increases the concentration of SAM in rat brain (Baldessarini, Biochem. Pharmacol., 15:741-748, 1966), and folate and B12 deficiencies lower brain SAM in rats (Bottiglieri et al., Drugs 48:137-152, 1994). Consistent with methionine supplementation increasing SAM generation, the average daily intake of methionine does not saturate MAT (Bottiglieri et al., Drugs 48:137-152, 1994). The one-carbon cycle is shown below.

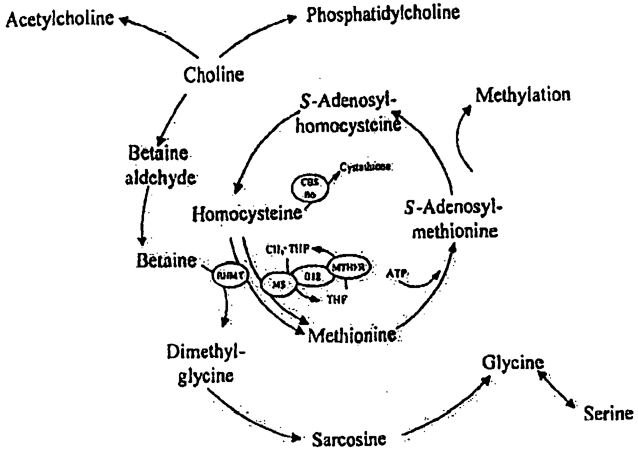

There is an alternate pathway for the conversion of homocysteine to methionine in which betaine is the methyl donor, catalyzed by betaine-homocysteine methyltransferase (BHMT). The efficacy of this alternate pathway is revealed by the ability of betaine to effectively lower the very high homocysteine levels seen with cystathione-β-synthase (CBS) deficiency (Wilcken et al, N. Eng. J. Med., 309:448-453, 1983, Wilcken et al, Ann. NY. Acad. Sci, 854:361-370, 1998); CBS converts homocysteine to cysteine (cystathione). Betaine has been shown to function as a methyl donor in mammalian tissues (Baldessarini, Int. Rev. Neurobiol, 18:41-67, 1975), and increase SAM (Abdelmalek et al, Am. J. Gastrenterol, 96:2711-2717,2001). SAM has been found effective in the treatment of moderate depression. In general, SAM is well tolerated, with the most *frequent* adverse effects for the oral supplement being mild to moderate gastrointestinal symptoms. However, SAM is an unstable compound, with poor bioavailability and a short plasma half-life. The vast majority of studies of SAM efficacy in depression have used parenteral administration, iv or im, usually 2 or 3 times per day. Additionally, SAM causes accumulation of homocysteine and depletion of folate (Loehrer et al, Clin. Sci., 91:79-86, 1996), which could have clinically adverse consequences. The composition and methods described herein avoid these adverse effects.

An alternative to administering exogenous SAM is to augment its endogenous synthesis. WO96/33703 relates to the concept of improving methylation reactions in neurotransmitter metabolism in order to treat depression, and concludes that methyl donors involved in one carbon metabolism may have a major role in modulation of neurotransmitter levels and, therefore, in the treatment of depression. WO 001/25764 teaches that betaine acts as a methyl donor in one-carbon metabolism.

Administration of L-methionine, betaine and folate or folic acid augments SAM synthesis. Betaine and folate or folic acid also mitigate possible unwanted effects of exogenous L-methionine, most importantly accumulation of homocysteine, which is also caused by exogenous SAM. L-methionine and betaine are orally active, generally safe compounds with putative nervous and digestive systems benefits. Folate supplements have long been used safely and effectively for conditions associated with folate depletion. A regimen of L-methionine, betaine and folate or folic acid produces physiological and clinical effects similar to parenteral SAM, and easier to use in the actual clinical treatment of depression.

Augmenting the one-carbon cycle, however, can also have negative side effects. For example, SAM, methionine, and betaine can each worsen schizophrenia. SAM can also induce hypomania or mania in bipolar patients. Methionine can cause large increases in homocysteine in patients with renal insufficiency or homocystinuria, and hyperhomocysteinemia is a cardiovascular risk.

### The One-Carbon Cycle & Affective Disorders

Disturbances in the one-carbon cycle have been implicated in affective disorders. MAT catalyzes the synthesis of SAM from L-mothionine and ATP, and unipolar depressives have been found to have significantly lower MAT activity in their erythrocytes than healthy controls (Alarcon et al., J. Affect Dis., 9:297-301, 1985). The regeneration of SAM is mediated by folate derivatives (Reynolds et al., Lancet, 28:197-198, 1984), and the most common neuropsychiatric disorder associated with folate deficiency is depression (Reynolds EH In Clinics in Haematology (ed by AV Hoffbrand), pp 661-696, Saunders, London, 1976, Botez et al., Nature, 278:182-183, 1979, Shorvon et al., Brit. Med J., 281:1036-1042, 1980, Alpert et al., Nutr. Rev., 55:145-149, 1997). Interestingly, high levels of folate are found in synaptic regions (McClain et al., J. Neurochem., 24: 719-722, 1975), and folate deficiency leads to decreased brain serotonin in man and the rat (Botez et al., Nature, 278:182-183, 1979). One-carbon cycle involvement in affective disorders is further supported by reports that folate augments antidepressant response rates (Cimino et al., Life Sci., 34:2029-2039, 1984, Coppen et al., J. Affect. Disord., 10:9-13,1986, Coppen et al., J. Affect. Discord., 60:121-130, 2000; Alpert et al., Ann. Clin. Psychiatry, 14:33-38, 2002), that methyltetrahydofolate may have clinical antidepressant activity (Guaraldi et al., Ann. Clin. Psychiatr., 5:101-105, 1993), and that SAM is effective in the treatment of depression.

### S-adenosylmethionine & Depression

SAM was found effective in open studies as a treatment for depression (Belsanti et al., Folia Neuropsychiatry, 16:651-653, 1973, Fazio et al., Int. Res. Commun. Syst., 2:1015, 1974, Calandra et al., Minerva Psychiatr., 20:147-152, 1979, Salvadorini et al., Curr. Ther. Res., 27:908-918, 1980, Agnoli et al., Clin. Neuropharmacol., 7(suppl): 104-105, 1984, Lipinski J et al., Am. J. Psychiatry, 141:448-450, 1984, Rosenbaum J et al.,: Acta Psychiatr Scand., 81:432-436, 1990, Fava et al., Psychiatry Res., 28:295-297, 1995). Additionally, in the treatment of depression SAM was shown in double-blind studies to be superior to placebo (Fazio et al., Minerva Med., 64:1515-1529, 1973, Agnoli et al., Clin. Ther., 75:567-579, 1975, Agnoli et al., J. Psychiat. Res., 13:43-54, 1976, Barberi et al., Minerva Psichiatr., 19:235-239, 1978, Muscettola et al., In Typical and Atypical Antidepresants: Clinical Practice (ed by E Costa, G Racagni), pp 151-156, Raven, New York, 1982, Caruso et al., Lancet, I:904, 1984, Carney et al., Clin. Neuropharmacol,. 9:379-385, 1986, Kagan et al., Am. J. Psychiatry., 147:591-595, 1990, Salmaggi et al., Psychother. Psychosom.. 59:34-40, 1993), and equal to clomipramine (Miccoli et al., Acta. Neurol., (Napoli) 33:243-255, 1978, Del Vecchio et al., Riv. Sperimentale Freniatria, 102:344-358, 1978, Miccoli et al., Acta. Neurol., (Napoli) 33:243-255, 1978, Scarzella et al., Riv. Sperimentale Freniatria, 102:359-365, 1978, Calandra et al., Minerva Psychiatr., 20:147-152, 1979, Kufferle et al., In Typical and Atypical Antidepresants: Clinical Practice (ed by E Costa, G Racagni), pp 175-180, Raven, New York, 1982, Giner Ubago et al., Actas. Luso-Esp. Neurol. Psiquitr., 12:73-80, 1984), imipramine (Mantero et al., Minerva Med., 66:4098-4101, 1975), desipramine (Bell et al., Am. J. Psychiatry, 145:1110-1114, 1988), amitriptyline (Monaco et al., Riv. Neurol., 49:417-439, 1979) and nomifensine (Scaggion et al., Minerva Psichiatr., 23:93-97, 1982). SAM was reported to have a more rapid onset of action than standard antidepressants, with improvement in the first week (Miccoli et al., Acta. Neurol., (Napoli) 33:243-255, 1978, Salvadorini et al., Curr. Ther. Res., 27:908-918, 1980, Bell et al., Am. J. Psychiatry, 145:1110-1114, 1988). Additionally, only mild and transient adverse effects have been reported, such as flatus, nausca, constipation and loose stools (Kagan et al., Am. J. Psychiatry., 147:591-595, 1990, Salmaggi et al., Psychother. Psychosom., 59:34-40, 1993). Healthy controls were unable to distinguish acute infusions of SAM from placebo (Sherer et al., Psychiatry Res., 17:111-118, 1986). However, consistent with the reported antidepressant properties of SAM, induction of hypomania or mania has been reported (Lipinski J et al., Am. J. Psychiatry, 141:448-450, 1984, Kagan et al., Am. J. Psychiatry., 147:591-595, 1990), especially in bipolar patients (Carney et al., Br. J. Psychiatry, 154:48-51, 1989).

SAM effects in depression may be due to its effects on brain monoamine levels. SAM increased the dopamine metabolite HVA and the serotonin metabolite 5-HIAA in the CSF of depressed patients (Agnoli et al., In Depressive Disorders (ed G Garattini) pp 447-458, Schattauer-Verlag, Stuttgart, 1977, Bottiglieri et al., J. Neurol. Neurosurg. Psychiatry 53:1096-1098, 1990, Bell et al., Acta. Neurol. Scand., 154:15-18, 1994), and increased turnover of serotonin and norepinephrine in rat brain (Botez et al., Nature, 278:182-183, 1979). SAM also augmented the antidepressant effects of β-adrenergic agonists in depressed patients (Manna et al., Proceedings of the 13th C.I.N.P. Congress, Jerusalem, Israel, 1982) and in an antidepressant screen in rats (Dunn et al., Soc. Neurosci. Abstr., 14:45, 1988). Neuroendocrine studies also indicate indicate enhancement of dopaminergic systems by SAM, with inhibition of secretion of prolactin (Thomas et al.,. Int. Clin. Psychopharmacol., 2: 97-102, 1987, Fava et al., J. Psychiatr. Res., 24:177-184, 1990) and thyrotropin (TSH) (Fava et al., J. Psychiatr. Res., 24:177-184, 1990) by SAM. SAM effects on monoamines may be mediated by altering transmethylation reactions for which SAM is the methyl donor, and by increased phospholipid methylation leading to decreased membrane fluidity and increased receptor-effector coupling (Baldessarini, Int. Rev. Neurobiol., 18:41-67, 1975, Cimino et al., Life Sci., 34:2029-2039, 1984, Reynolds et al., Psychological Medicine, 13:705-710, 1983, Fava et al., J. Psychiatr. Res., 24:177-184, 1990, Bottiglieri et al., Drugs 48:137-152, 1994).

Studies with SAM in depression have noted no or only mild and transient adverse effects (Miccoli et al., Acta. Neurol., (Napoli) 33:243-255, 1978, Salvadorini et al., Curr. Ther. Res., 27:908-918, 1980, Kufferle et al., In Typical and Atypical Antidepresants: Clinical Practice (ed by E Costa, G Racagni), pp 175-180, Raven, New York, 1982, Muscettola et al., In Typical and Atypical Antidepresants: Clinical Practice (ed by E Costa, G Racagni), pp 151-156, Raven, New York, 1982, Lipinski J et al., Am. J. Psychiatry, 141:448-450, 1984, Bell et al., Am. J. Psychiatry, 145:1110-1114, 1988, Rosenbaum J et al.,: Acta Psychiatr Scand., 81:432-436, 1990, Kagan et al., Am. J. Psychiatry., 147:591-595, 1990, Salmaggi et al., Psychother. Psychosom., 59:34-40, 1993, Fava et al., Psychiatry Res., 28:295-297, 1995). The most frequent adverse effects for oral SAM were gastrointestinal symptoms and mild anxiety. Salvadorini et al., Curr. Ther. Res., 27:908-918, 1980, found no effects on EEG, EKG and laboratory measures by SAM given *iv* or *im* to depressed patients. Kufferle et al., In Typical and Atypical Antidepresants: Clinical Practice (ed by E Costa, G Racagni), pp 175-180, Raven, New York, 1982, tested total protein, inorganic phosphate, calcium, BUN, creatinine, total bilirubin, alkaline phosphatase, triglycerides, LDH, SGPT (ALT), SGOT (AST), GGT, cholesterol, glucose, uric acid, sodium, potassium, chloride and CBC in 20 depressed patients receiving *i.v.* SAM; the only changes were minimal transient increases in SGPT and SGOT. Lipinski J et al., Am. J. Psychiatry, 141:448-450, 1984, found no effects on blood pressure, heart rate or respirations by SAM in depressed patients. Sherer et al., Psychiatry Res., 17:111-118, 1986, reported that daily SAM infusion (*iv*) for 7 days in healthy volunteers was associated with decreases in standing, but not supine, heart rate (6 beats/minute); no dizziness or behavioral effects were reported by subjects, and in fact could not distinguish SAM infusion from placebo. Frezza et al., Hepatology, 4:274-278, 1984, reported that SAM decreased AST, ALT, conjugated bilirubin and total bile acids, and increased alkaline phosphatase, in 18 pregnant women with intrahepatic cholestasis of pregnancy. SAM was also well tolerated by 13 depressed Parkinson's Disease patients (Di Rocco et al., Movement Disorders, 15:1225-1229, 2000). Two patients left the study due to increased anxiety; one patient became manic and had pre-existing bipolar disorder, and the other patient's anxiety did not resolve with stopping SAM and was thought not related to the SAM. Mild, spontaneously resolving diarrhea or nausea was observed in 3 other patients who remained in the study; worsening of orthostatic hypotension was *not* noted in these Parkinson's Disease patients (Di Rocco et al., Movement Disorders, 15:1225-1229, 2000).

The vast majority of the SAM studies (26 of 30) used parenteral administration (*iv or im*); there are many fewer reports (4 studies) of SAM efficacy when administered orally (open = Rosenbaum J et al.,: Acta Psychiatr Scand., 81:432-436, 1990; controlled = Kagan et al., Am. J. Psychiatry., 147:591-595, 1990, Bell et al., Am. J. Psychiatry, 145:1110-1114, 1988, Salmaggi et al., Psychother. Psychosom., 59:34-40, 1993). Very few reports of efficacy in depression with oral SAM have been completed other than an open label study in 13 depressed Parkinson's disease patients (Di Rocco et al., Movement Disorders, 15:1225-1229, 2000), whose positive results are difficult to interpret since L-methionine may benefit Parkinson's disease itself (Smythies, Biol. Psychiatry, 19:755-758, 1984), a controlled study in which patients received oral SAM and another set of patients received imipramine and no difference in efficacy was determined. Another trial found that SAM augmented antidepressants in partial or non-responders (Alpert et al., Ann. Clin. Psychiatry, 14:33-38, 2002). The lack of results demonstrating the efficacy of orally administered SAM, or of common use of oral SAM in depressed patients leads to questions about the viability of SAM as a treatment for depression. SAM has proven to be an unstable compound with poor bioavailability, which is subject to a high first pass effect and has a short plasma half life demonstrating that SAM does not have the efficacy for a viable treatment of depression.

### Methionine

Methionine is an essential nonpolar amino acid and a lipotrophic agent. The recommended daily allowance of methionine is 2 grams per day. Although mammals cannot synthesize methionine, it is still utilized in a variety of biochemical pathways including being converted to SAM by methionine adenosyltransferase. SAM serves as a methyl donor in methyltransferase reactions and is converted to S-adenosylhomocysteine (SAH) and adenosylhomocysteinase converts SAH to homocysteine. Homocysteine can then be used to regenerate methionine by methionine synthase or it homocysteine can be remethylated using glycine betaine to methionine via the enzyme betaine-homocysteine methyltransferase. Homocysteine may also converted to cysteine. Elevated levels of homocysteine may increase a person's risk of heart disease and stroke. Additionally homocysteine may damage coronary arteries or allow clots to more easily form.

### Folate

Folate is the anion form of folic acid, which are both forms of the water-soluble Vitamin B9. Both occur naturally in food and can be taken as a supplements. Folate is an essential micronutrient that is necessary for the production and maintenance of new cells, and is especially important during periods of rapid cell division and growth. The recommended daily allowance of folate is 400 micrograms per day.

Folate is also needed to replicate DNA, which helps prevent changes in DNA that could lead to cancer. Folate is in the form of series tetrahydrofolate compounds and its derivatives are substrates in a number of single-carbon transfer reactions and are also involved in the synthesis of dTh4P (2'-deoythymidine-5'-phosphate) from dUMP (2'-deoyuridine-5'-phosphate). Additionally folate helps convert vitamin B12 to one of its coenzyme forms and folate is known to augment antidepressant drugs. Low concentrations of folate, vitamin B12, or vitamin B6 increase the levels of homocysteine in blood. Clinical studies found that folate 0.5 to 5 mg/day prevented elevation of homocysteine levels by methionine loading (van Guldener et al., J. Int. Med., 245:175-183, 1999, van der Griend et al., Vasc. Med., 7:29-33, 2002).

### Betaine

Betaine is a natural oxidation product of choline and is contained in the average diet but not known to be needed. Betaine rich diets may reduce cardiovascular risk by reducing levels of homocysteine. Betaine functions closely with SAM, folic acid, and vitamins B6 and B12 to break down homocysteine and reduce toxic levels in the blood stream. The recommended daily allowance of betaine is 2 grams per day.

### L-methionine and Betaine vs. SAM

The formation of SAM involves addition of an adenosyl moiety to the sulfur group in methionine, positively charging the sulfur group and activating the methyl group. This activated methyl group in SAM is unstable, especially in gastric acids. Oral studies of SAM apparently awaited introduction of reportedly more stable SAM formulations, such as the toluene sulfate (Fetrow et al., The Annals of Pharmacotherapy, 35:1414-1425, 2001). The long-term safety of the stabilizing additions to oral SAM preparations is unclear. Other researchers have utilized enteric coating to address the difficulty of SAM instability (Rosenbaum J et al.,: Acta Psychiatr Scand., 81:432-436, 199). However, SAM is also rapidly metabolized by the liver, with a high first-pass effect (Stramentinoli et al., J. Pharmaco/. Exp. Ther., 209:323-326, 1979). Thus, parenteral studies with SAM typically administered 50-200 mg/day, while oral studies used 1600 mg/day or more.

One solution to the issue of the relative instability of SAM orally is to administer oral methionine instead. In healthy controls, a single oral dose of 400 mg of SAM was observed to have a half-life of 1.7 ± 0.3 hours, while oral methionine loading (100mg/kg) exhibited a half-life for increased SAM of 7.1 ± 3.9 hours (Loehrer et al., Clin. Sci., 91:79-86, 1996). Parentally administered SAM increases SAM levels in cerebral spinal fluid to about 130% of controls (Bottiglieri et al., J. Neurol. Neurosurg. Psychiatry 53:1096-1098, 1990), but intraperitoneally (*ip,* an enteral route) administered methionine was also found in rats to increase SAM in many tissues, including increasing SAM in brain to about 130% of controls (Baldessarini, Biochem. Pharmacol., 15:741-748, 1966, Lombardini et al., Molecular Pharmacol., 9:542-560, 1973). Folate supplementation would be needed to maintain elevated SAM levels from chronic methionine administration since 8 grams/day of L-methionine for 4 days in healthy controls decreased serum folate levels (Connor et al., Post Grad. Med., 54:318-320, 1978) and folate deficiency lowered brain SAM in rats (Bottiglieri et al., Drugs 48:137-152, 1994).

Another approach to increase methyl groups available in the brain is to augment SAM levels by endogenous synthesis, i.e. to administer L-methionine and betaine rather than administering exogenous SAM for the treatment of depression. Betaine is trimethylglycine, and can function as a methyl donor in mammalian tissues (Baldessarini, Int. Rev. Neurobiol., 18:41-67, 1975) and increase SAM (Abdelmalek et al., Am. J. Gastrenterol., 96:2711-2717, 2001). Betaine is orally active and safe, albeit central activity is indicated for betaine by its exacerbatation of schizophrenia (Brune et al., Recent Advances in Biol. Psychiatry, 5:144-160, 1963). L-methionine and betaine augmented the antidepressant-like effects in rats of the β-agonist clenbuterol on an operant screen for antidepressant drugs, the DRL 72-S schedule (Seiden, et al., Psychopharmacol., 86:55-60, 1985, Dunn et al., Soc. Neurosci. Abstr., 14:45, 1988); the augmentation associated with betaine was extremely robust. SAM was found to augment the clinical antidepressant activity of imipramine (Berlanga et al., Psychiatry Res., 44:257-262 , 1992) and a β-adrenergic agonist (Manna et al., Proceedings of the 13th C.I.N.P. Congress, Jerusalem, Israel, 1982), and SSRIs and venlafaxine (Alpert et al., Ann. Clin. Psychiatry,14:33-38, 2002).

Treatment with methyl donors been reported to benefit several neuropsychiatric illnesses, and has been associated with remyelination (Bottiglieri et al., Drugs 48:137-152, 1994). Preliminary clinical studies suggest that L-methionine may benefit Parkinson's disease (Smythies et al., South Med. J., 77:1577, 1984) and AIDS myelopathy (Di Rocco et al., Neurology, 51:266-268, 1998). Betaine is being investigated as treatment for Alzheimer's disease (Knopman et al., Alzheimer Dis. Assoc. Disord., 15:162-165, 2001) and Rett's Syndrome (combined with folate, Percy Clinical trials and treatment prospects. Ment. Retard Dev. Disabil. Res. Rev., 8:106-111, 2002). Clinical studies have also demonstrated efficacy for betaine in the treatment of homocystinuria (Wilcken et al.. N. Eng. J. Med., 309:448-453, 1983, Wilcken et al., Ann. NY. Acad. Sci., 854:361-370, 1998) and non-alcoholic steatohepatitis (Miglio et al., Arzneimittelforschung, 50:722-727, 2000, Abdelmalek et al., Am. J. Gastrenterol., 96:2711-2717, 2001).

Clinical reports indicate that L-methionine should not be given to patients with schizophrenia, and not to patients with bipolar disorder or history of mania. Clinical reports also indicate that, due to induction or worsening of hyperhomocysteinemia, L-methionine should not be given to patients with homocystinuria or renal failure, and not given to patients with cardiovascular disease. Other than in schizophrenic and bipolar patients, betaine has minimal or no adverse effects, with one study reporting no differences from placebo (Miglio et al., Arzneimitte/forschung, 50:722-727, 2000), and another study reporting only transient and tolerable nausea or loose stools (Abdelmalek et al., Am. J. Gastrenterol., 96:2711-2717, 2001). Folate has long been used safely for conditions related to folate deficiency. Oral methionine loading (Pollin et al.,. Science, 133:104-105, 1961, Heath et al., Arch. Gen. Psychiatry, 14:213-217, 1966, Spaide et al., Biol. Psychiatry, 1:227-33, 1969, Spaide J et al., Am. J. Clin. Nutr., 24:1053-1059, 1971, Ananth et al., Can. Psychiatr. Assoc. J., 15:15-20. 1970, Antun et al., J. Psychiatr. Res., 8:63-71, 1971; see Cohen et al., Biol. Psychiatr., 8:209-225, 1974 for review), oral betaine (Brune et al., Recent Advances in Biol. Psychiatry, 5:144-160, 1963) and parenteral SAM (Fazio et al., Int. Res. Commun. Syst., 2:1015, 1974) have been found to exacerbate psychosis in schizophrenic patients. For methionine, administration of 10-20 grams/day worsened psychosis in about 40% of schizophrenic patients receiving it (Cohen et al., Biol. Psychiatr., 8:209-225, 1974). Psychosis has been also associated with homocystinuria (Baldessarini, Int. Rev. Neurobiol., 18:41-67, 1975), which has very high serum methionine levels. These data suggest that L-methionine, betaine and SAM can enhance brain dopaminergic activity because central hyperdopaminergic states have been associated with psychosis. Consistent with this hypothesis, L-methionine (Smythies et al., South Med. J., 77:1577, 1984) and SAM (Di Rocco et al., Movement Disorders, 15:1225-1229, 2000) have been found to benefit Parkinson's Disease, and CSF and neuroendocrine clincial studies indicate enhancement of dopaminergic systems by SAM (Fava et al., J. Psychiatr. Res., 24:177-184, 1990). L-methionine, betaine and SAM may similarly be able to benefit depression because hypodopaminergic function has been postulated in depression (Fawcett et al., In Textbook of Psychopharmacology, 2nd ed (ed AF Schatzberg, CB Nemeroff), pp.503-522, American Psychiatric Press, Washington, DC, 1998).

L-methionine and betaine should not to be administered to patients with bipolar disorder or history of mania. Methionine and betaine supplementation increase SAM in mammalian tissues (Baldessarini, Biochem. Pharmacol., 15:741-748, 1966, Baldessarini, Int. Rev. Neurobiol., 18:41-67, 1975, Abdelmalek et al. Am. J. Gastrenterol., 96:2711-2717, 2001), and SAM can induce hypomania or mania (Lipinski J et al., Am. J. Psychiatry, 141:448-450, 1984, Kagan et al., Am. J. Psychiatry., 147:591-595, 1990), especially in bipolar patients (Carney et al., Br. J. Psychiatry, 154:48-51, 1989).

L-methionine should not be given to patients for whom induction or worsening of hyperhomocysteinemia has known or probable adverse consequences, and so should definitely not be given to patients with homocystinuria or renal failure, and probably not given to patients with cardiovascular disease. The three most important causes of hyperhomocysteinemia are genetic abnormalities in homocysteine metabolism, certain vitamin deficiencies, and renal failure (van Guldener et al., J. Int. Med., 245:175-183, 1999). The most common genetic abnormality in homocysteine metabolism is cystathione-β-synthase (CBS) deficiency (Lehninger Principles of Biochemistry, Worth, New York, 1982, Mudd et al.,. In Cecil Textbook of Medicine, 18th Edition (ed. by JB Wyngaarden and LH Smith), pp 1160-1161, WB Saunders, Philadelphia, 1988). CBS converts homocysteine to cysteine (cystathione), with pyridoxine (vitamin B6) as a cofactor (Van Guldener et al 1999). CBS deficiency produces very high homocysteine levels in blood (> 80 µmol/L; normal fasting 3-12 µmol/L) and consequent homocystinura. CBS has a prevalence of 1 in 200,000 live births, and patients with CBS deficiency suffer from mental retardation (median IQ=78), osteoporosis and bony abnormalities, ectopia lentis and thromboembolic complications (Mudd et al.,. In Cecil Textbook of Medicine, 18th Edition (ed. by JB Wyngaarden and LH Smith), pp 1160-1161, WB Saunders, Philadelphia, 1988). High doses of pyridoxine have been used with some success to treat homocystinura (Mudd et al.,. In Cecil Textbook of Medicine, 18th Edition (ed. by JB Wyngaarden and LH Smith), pp 1160-1161, WB Saunders, Philadelphia, 1988).

The loss of the labile methyl group from SAM yields S-adenosylhomocysteine, which is hydrolyzed to homocysteine (Lehninger Principles of Biochemistry, Worth, New York, 1982). Homocysteine is removed by two pathways (van Guldener et al., J. Int. Med., 245:175-183, 1999): irreversible transsulfuration to cysteine (cystathione) by cystathione-β-synthase (CBS; requires vitamin B6), and by remethylation to methionine. This remethylation can occur by 2 routes, as mentioned above. Again, these arc conversion to S-adenosylhomocysteine by methionine synthase (aka homocysteine methyltransferase; requires methylcobalamine), and conversion to methionine by betaine-homocysteine methyltransferase (BHMT; requires zinc). Methylcobalamine is generated from vitamin B12 in reactions requiring folate derivatives.

Moderate hyperhomocysteinemia (normal fasting 5-15 µmol/L per van Guldener et al., J. Int. Med., 245:175-183, 1999) is considered an independent risk factor for coronary artery disease for patients under age 46 (Reis et al., Coron. Artery Dis., 6:851-856, 1995, van der Griend et al., Vasc. Med., 7:29-33, 2002). Homocysteine has been postulated to exert its adverse cardiovascular effects by injury to endothelial cells (Nygard et al., N. Engl. J. Med., 337:230-236, 1997).

Exogenous SAM and L-methionine increase homocysteine (Loehrer et al., Clin. Sci., 91:79-86, 1996). Methionine loading, generally 100 mg/kg body weight, has been used to detect the up to 50% of patients with abnormal homocysteine metabolism who do not have elevated fasting homocysteine (van der Griend et al., Vasc. Med., 7:29-33, 2002). Post-methionine loading homocysteine levels above 38 µmol/L may be a threshold indicating cardiovascular risk, however, the clinical utility of the methionine loading test is unclear (van der Griend et al., Vasc. Med., 7:29-33, 2002). No change in post-load homocysteine nor methionine clearance from methionine loading tests performed before and after 2 weeks of excessive daily methionine (300% of normal, about 6 grams per day) was found in 6 healthy subjects (Andersson et al., Clin. Chim. Acta., 192:69-76, 1990). A study that followed 887 patients for 30 days following a single methionine loading test concluded that it is a safe test, with only transitory adverse effects, the most common being dizziness, sleepiness, nausea, polyuria, and increased or decreased blood pressure (Krupkova-Meixnerova et al., Clin. Nutr., 21:151-156, 2002); no deaths occurred.

Clinical studies found that folate 0.5 to 5 mg/day prevented elevation of homocysteine levels by methionine loading (van Guldener et al., J. Int. Med., 245:175-183, 1999, van der Griend et al., Vasc. Med., 7:29-33, 2002). Conversely, chronic methionine loading, 8 grams/day for 4 days in healthy controls, decreased serum folate levels (Connor et al., Post Grad Med., 54:318-320, 1978). Vitamin B6 deficiency in rats worsened post-methionine loading homocysteine levels (Miller et al., Am. J. Clin. Nutr., 59:1033-1039, 1994). However, in clinical studies of folate supplementation, the addition of vitamin B6 did not further decrease post-methionine loading homocysteine levels (van der Griend et al., Vasc. Med., 7:29-33, 2002). Interestingly, oral betaine at 4 grams/day in chronic renal failure patients, who also received 5 mg/day folate and 50 mg/day vitamin B6, attenuated elevation of homocysteine levels by methionine loading (McGregor et al., Kidney Int., 61:1040-1046, 2002). Betaine at 500 mg/kg orally in rabbits was even reported to decrease serum and hepatic cholesterol and triglycerides, leading to suggestions that it may be an antisclerotic agent (Panteleimonova et al., Farmako/. Toksikol., 46:83-85,1983).

Naturally occurring vitamin B6 (pyridoxine) deficiency is rare (Smith et al., Principles of Biochemistry: Mammalian Biochemistry. 7th Edition. McGraw-Hill, NY, 1983). Isoniazide, iproniazide and phenelzine can deplete vitamin B6. Vitamin B6 supplementation is used to treat the hyperhomocysteinemia of CBS deficiency, essentially to increase the activity of any remaining CBS; CBS deficiency, is revealed by its elevated fasting homocysteine levels. Importantly, in clinical studies of folate supplementation, the addition of vitamin B6 did not further decrease post-methionine loading homocysteine levels (van der Griend et al., Vasc. Med., 7:29-33, 2002). However, the addition of betaine (4 grams daily) further decreased post-methionine loading (but not fasting) homocysteine levels by 18% in chronic renal failure patients receiving 5 mg folate and 50 mg pyridoxine daily (McGregor et al., Kidney Int., 61:1040-1046, 2002), so there is another reason to include betaine in the regime.

### Methionine, Betaine and Folate

L-methionine, betaine and folate or folic acid are generally safe agents. Folate or folic acid are included in the regimen to mitigate folate depletion by L-methionine. Folate or folic acid and betaine are included in the regimen to mitigate any hyperhomocysteinemia and increased cardiovascular risk.

L-methionine and betaine are compounds which augment the one-carbon cycle. L-methionine is an essential amino acid. Betaine (trimethylglycine) is a natural oxidation product of choline (Stryer Blochemistry, WH Freeman. San Francisco, 1981) and about 0.5 to 2 grams per day is contained in an average diet (Olthof et al., J. Nutr., 133:4135-4138, 2003). It is used as a dietary supplement by some people, but is not known to be needed in the diet. A betainerich diet may reduce cardiovascular risk (Olthof et al., J. Nutr., 133:4135-4138, 2003). Folate (vitamin B9) is an essential micronutrient (daily requirement = 400 mcg., Lehninger Principles of Biochemistry, Worth, New York, 1982) and its derivatives are required by the one-carbon cycle.

The efficacy and tolerability in unipolar depression of one-carbon cycle augmentation with L-methionine, betaine and folate are discussed below in the Examples. Efficacy is evaluated for: 1) time course; 2) response rate at study end-point. Tolerability is formally evaluated throughout the study. The importance of this study derives from the suffering inherent in the delayed effects of antidepressant drugs, and the 10-15% of depressed patients refractory to or intolerant of existing therapies (Kaplan et al., Comprehensive Textbook of Psychiatry, 6th ed, Williams and Wilkins, Baltimore, 1995).

Disturbances in the one-carbon cycle have been implicated in affective disorders and agents augmenting the one-carbon cycle have antidepressant effects. The one-carbon cycle involves the generation of S-adenosylmethionine (SAM), the utilization of the labile methyl group of SAM in transmethylation reactions, and the regeneration of SAM. SAM has been found effective in the treatment of depression, especially with parenteral administration. An alternative to administering exogenous SAM for the treatment of depression would be to augment its endogenous synthesis. A regimen of L-methionine, betaine and folate or folic acid augments SAM levels better than exogenous SAM, and avoid the clinically adverse consequences from the accumulation of homocysteine and depletion of folate. The efficacy and tolerability in unipolar depression of a regimen of L-methionine, betaine and folate is evaluated. The efficacy of the regimen is be evaluated for time course and response rate at study end-point. Tolerability is also evaluated.

Methionine is safe in appropriate populations and should be avoided in others. For example, L-methionine is not to be administered to patients with schizophrenia, bipolar disorder, renal failure or homocystinuria, and not to those with cardiovascular disease.

Risk of psychosis and mania from L-methionine and betaine is reduced by screening out patients with a history of psychosis or mania, such as psychotic depression, schizophrenia, bipolar or schizoaffective disorder. Additionally, symptoms of psychosis and mania are evaluated during treatment with use of the BPRS and YMRS. Generally, most studies finding worsening of schizophrenia with methionine loading had co-administered an MAO inhibitor to enhance the potential production of postulated transmethylated psychotogens.

Causes of morbidly and mortality in patients with homocystinuria (which has very high serum homocysteine) are thrombotic and thromboembolic; patients with homocystinuria. No thromboic and thromboembolic complications have been noted in any study of SAM or methionine therapy.

Hyperhomocysteinemia is an independent risk factor for coronary artery disease and the study regimen may potentially increase serum homocysteine, despite the inclusion in the study regimen of betaine and folate or folic acid to prevent increases in serum homocysteine from L-methionine.

### Clinical Efficacy

Time of course of clinical improvement for the regimen of L-methionine, betaine and folate or folic acid is evaluated. Additionally, measurement of depressive symptoms at frequent intervals during the study is used to evaluate the time course of clinical improvement. Moreover, the clinical response rate for the regimen of L-methionine, betaine and folate or folic acid is measured.

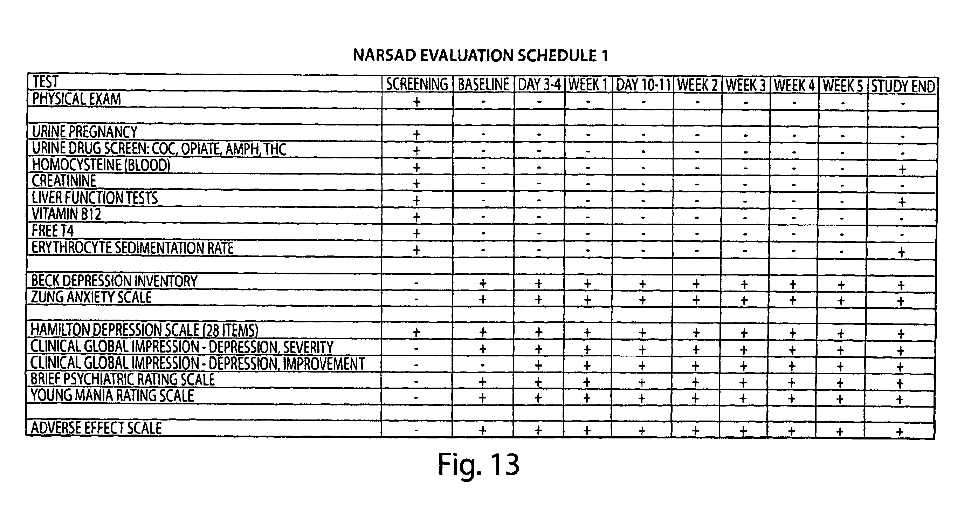

Measurement of depressive symptoms before and after 6 weeks treatment is used to generate comparisons to evaluate the clinical response rate. Tolerability of the regimen of L-methionine, betaine and folate or folic acid is also determined. Measurement of psychiatric and medical adverse effects at frequent intervals during the study, as well as lorazepam use and study drop-out rates, are used to evaluate tolerability. Additionally, homocysteine (blood) and liver function tests are measured at baseline and study end (see Figure 13: NARSAD 2004 Evaluation Schedule 1).

The importance of the drug combination derives from the suffering inherent in the delayed effects of antidepressant drugs, and the 10-15% of depressed patients refractory to or intolerant of existing therapies. Additionally, the further understanding of the role of the one-carbon cycle in unipolar depression and how the augmentation of the One-Carbon Cycle improves the depressive symptoms of the patients. The regimen also has minimal adverse effects. Thus, the drug combination therapy improves the treatment of unipolar depression.

The compositions and methods were evaluated as follows:

The treatment trial is carried out for a duration of 6 weeks. Six week trials are commonly utilized in assessing psychiatric medications as many agents require at least two weeks to begin to yield improvements, and patients may often continue to improve up to six weeks into the treatment. Longer trials subject non-responders to possibly ineffective therapy and incur greater expense. Shorter trials risk missing slow responders and are more confounded by spontaneous improvement of mood.

The oral regimen studied was 5 grams of L-methionine (10 capsules) + 10 grams of betaine (10 tablets) + 5 micrograms of folate (1 tablet) (total = 10 capsules + 11 tablets daily). Patients are permitted to take the 21 pills per day with or without food, at any time of day, in single or divided doses, and can mix them in liquids, shakes or foods. Starting doses are fixed in individual patients, but decreases in dose are permitted if continued adverse effects would lead to loss of the subject from the study.

Dispensing of medication was performed using known methods. The L-methionine, betaine and folate are purchased from a reputable nutritional supplement vendor and stored in controlled conditions by the CHA Pharmacy. The maximum strength available for L-methionine is 500 mg capsules, for betaine as 1000 mg tablets and folate as 5 mg tablets, thus the need for 21 pills per day. Patients are instructed to bring all pill bottles to each study visit and new pills are dispensed, so that pill counts can be performed to evaluate treatment adherence; statistical comparisons are made for treatment adherence for the treatment group.

### Outcome Measures and Statistical Analysis

Clinical outcome measures include clinician ratings and patient self ratings. Baseline scores are obtained for all scales before initiation of treatment. All rating scales are also completed twice weekly for the first 2 weeks, then once per week for weeks 3, 4, 5 and 6. Thus, rating scores for all scales are available for 9 time points. Last-Observation-Carried-Forward (LOCF) analyses are performed for all subjects taking at least one day of the L-methionine, betaine or folate. Completer analyses are also performed.

Vital signs and body weights are obtained at each study visit. Homocysteine levels, liver function tests (LFTs), lipid panels and electrocardiogams (EKGs) are obtained at baseline and endpoint.

Clinicians complete the Hamilton Depression Rating Scale 28 item version (HDRS-28), Montgomery-Asberg Depression Rating Scale (MADRS), Clinical Global Impression (CGI) scales for severity (S) and improvement (I) of depression, Brief Psychiatric Rating Scale (BPRS), Young Mania Rating Scale (YMRS), Hamilton Anxiety Scale (HAM-A), Liebowitz Social Anxiety Scale (LSAS), Clinical Global Impression (CGI) scales for severity (S) and improvement (I) of anxiety, UKU Side Effects Rating Scale, Arizona Sexual Experiences Scale (ASEX), Pittsburgh Sleep Quality Index (PSQI; Buysse et al., 1989), and Epworth Sleepiness Scale (ESS). HDRS-17 item scale scores are calculated from items 1-17 of the HDRS-28, and a BPRS psychosis subscale score is calculated from items 4, 7, 11, 12 and 15 of the BPRS. Patient self ratings include the Beck Depression Inventory (BDI) and Zung Anxiety Scale (ZAS). The CGI scales is used to enhance detection of clinical worsening (see Off-Study Criteria), as well as to evaluate efficacy. The BPRS psychosis subscale was used to enhance detection of psychotic symptoms, and the YMRS to enhance detection of manic symptoms.

### Example 1

The efficacy and safety of L-methionine, betaine and folate in unipolar depression were determined by evaluating the short-term effects of L-methionine, betaine and folate in the treatment of acute unipolar depression. S-adenosylmethionine (SAMe) is effective in the treatment of unipolar depression, and that methionine and betaine can increase SAMe in the brain.

An open-label, prospective, non-randomized, 6-week study of fixed doses was conducted in outpatients with unipolar depression. Patients received 10 grams of L-methionine, 10 grams of betaine and 5 mg of folic acid daily. Sample: unipolar patients with depressive symptoms. Some patients received an adjusted dose of 5 grams L-methionine daily. Patients were not on any psychotropic medications with the except of 0.5 mg of lorazepam daily. Clinicians completed the Hamilton Depression Rating Scale 28 Item (HAMD-28), and the Clinical Global Impression Severity and Improvement Scales (CGI-I, CGI-S) at each clinical visit. Patients completed the Beck Depression Inventory (BDI) at each clinical visit.

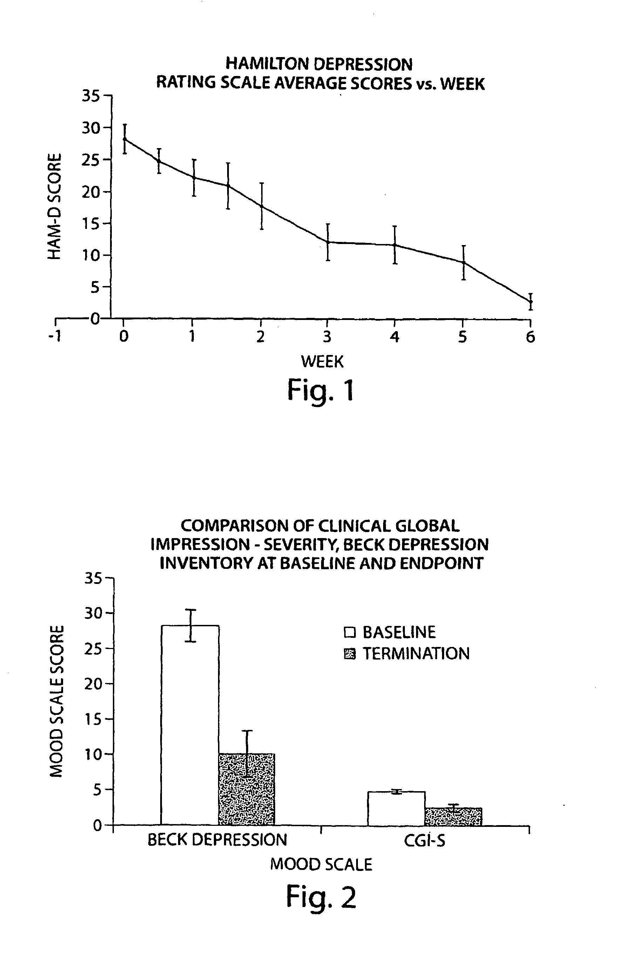

Independent t-test analyses were conducted for baseline and endpoint (LOCF) values. Analysis of Variance (ANOVA) were conducted on ratings scales in relationship to time. There was a significant decrease in depressive symptoms as scored on the HAM-D - (F(1,48) = 6.99, p<0.001). Post-hoc tests showed Weeks 2-6 scores as significantly decreased from baseline, where p<0.05 (Figure 1). There was a significant decrease in depressive symptoms as reported on the BDI (mean difference = -18.17, 95% CI [-25.89, -10.44]) (Figure 2). There was a significant decrease in depression illness severity as reported on the CGI-S (mean difference =-2.16, 95% CI [-3.56, -0.77]).

Side effects were reported but did not contraindicate use of the formulation. 5 of 7 subjects reported nausea (n=2), dizziness (n=2), and constipation (n=2) being the most common. Additional side effects reported included dry mouth, headaches, diarrhea, and stomach ache. Nausea and dizziness led to early termination for two subjects. There was no significant weight change from baseline to termination noted from the use of the three supplements (mean difference = -3.13 lbs, 95% CI [-6.90, 13.15]).

These data indicate that L-methionine, betaine and folate significantly reduced depressive symptoms.

### Example 2

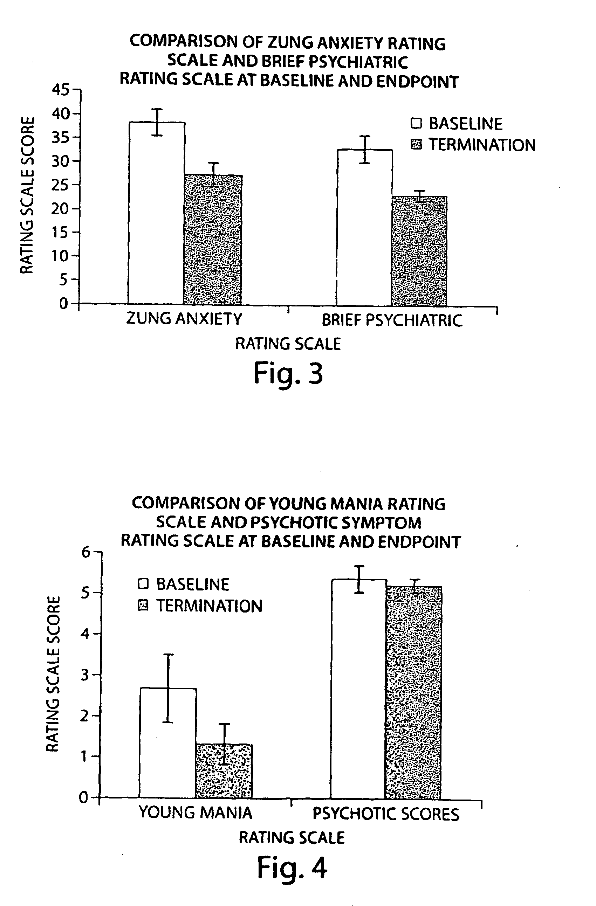

An open-label, prospective, non-randomized, 6-week study of fixed doses conducted in unipolar outpatients. Patients received 10 grams of L-methionine, 10 grams of betaine and 5 mg of folic acid daily. A subset of patients received an adjusted dose of 5 grams of L-methionine daily. The test population composed Unipolar patients with depressive symptoms. Patients were not on any psychotropic medications with the exception of 0.5 mg of lorazepam daily as needed. Clinicians completed Young Mania Rating Scale (YMRS), and Brief Psychiatric Rating Scale (BPRS) at each clinical visit. Patients completed the Zung Anxiety Scale (ZA) at each clinical visit. An additional Psychotic Symptom Rating Score (PSRS) was calculated from the summation of five items on the BPRS, including Conceptual Disorganization-Disorganized Speech, Mannerisms and Posturing, Suspiciousness, Hallucinatory Behavior, and Unusual Thought Content. Independent t-test analyses were conducted for baseline and endpoint (LOCF) values. An open label, prospective, non-randomized, 6-week study of fixed doses of methionine, betaine and folate, was conducted in depressed unipolar outpatients. There was a significant decrease in anxiety symptoms as reported on the ZA, (mean difference = 11.0, 95% CI [-17.83, - 4.17]) (Figure 3). There was a significant decrease in overall psychiatric symptoms as scored on the BPRS, (mean difference = - 9.83, 95% Cl [-17.21, -2.46]). There was no increase in manic symptoms as scored on the YMRS, (mean difference = -1.33, 95% CI [-3.70, 1.03] (Figure 4). There was no change in the five item PSRS from baseline to termination (mean difference =-0.17, 95% CI [-1.19, 0.87]).

As discussed above, side effects were reported in 5 of 7 subjects; nausea (n=2), dizziness (n=2), and constipation (n=2) being the most common. Other reported side effects included dry mouth, headaches, diarrhea, and stomach ache. Nausea and dizziness led to early termination for two subjects. There was no significant weight change from baseline to termination noted from the use of the three supplements (mean difference = -3.13, 95% CI [-6.90, 13.15]).

These data indicated that treatment with L-methionine, betaine and folic acid significantly decreased anxiety and overall psychiatric symptoms.

### Example 3

An open label, prospective, non-randomized, 6-week study of fixed doses of methionine, betaine and folate, was conducted in depressed unipolar outpatients. No other psychotropic medications were allowed. Hamilton Depression Rating Scale (HAMD) and Beck Depression Inventory (BDI) were administered to evaluate depressive symptoms. Clinical Global Impression Scale (CGI) and Brief Psychiatric Rating Scale (BPRS) were administered to evaluate overall psychiatric symptom severity. Patients were classified with either typical or atypical depression based upon a retrospective review of sleep and appetite symptoms (poor appetite/reduced sleep = typical, increased appetite/hypersomnia = atypical) at baseline. Preliminary analysis of 13 patients was conducted.

Psychiatric assessments were obtained for 5 men and 8 women (7 typical, 6 atypical). By ANOVA, scores improved for both typical and atypical patients on the HAMD (p=0.0003 and p<0.0001), BPRS (p=0.001 and p=0.0049) and CGI (p<0.0001 and pp.0002). Mean BDI scores improved significantly for atypical patients from baseline (23.6±1.5) to endpoint (8.4±3.3), where (t=3.17, p = 0.033), but not by ANOVA analysis (p=0.2593).

The combination of L-methionine, betaine and folate was found to improve acute unipolar depression in both atypical and typical depression patients.

### Example 4

The efficacy and safety of a combination treatment of L-methionine, betaine and folate in the treatment of unipolar depression with two different doses of L-methionine was conducted. The short-term effects of L-methionine, betaine and folate in the treatment of acute unipolar depression and the efficacy of the combination treatment with two different doses of L-methionine was evaluated.

An open label, prospective, non-randomized, 6-week study of fixed doses of 5g or 10g methionine, 10g betaine and 5mg folate, was conducted in depressed unipolar outpatients. No other psychotropic medications were allowed. Hamilton Depression Rating Scale (HAMD) and Beck Depression Inventory (BDI) were administered to evaluate depressive symptoms. Clinical Global Impression Scale (CGI) and Brief Psychiatric Rating Scale (BPRS) evaluated overall psychiatric symptom severity. Homocysteine (hCg) levels were drawn at baseline and endpoint. Following the completion of 5 patients, elevated hCg levels at termination led to a decrease in L-methionine doses from 10g daily to 5g daily. Analysis of 13 patients was conducted.

Psychiatric assessments were obtained for 5 men and 8 women (5 at 10g 1-methionine, 8 at 5g 1-methionine). By ANOVA, scores significantly improved with both 10g and 5g doses of 1-methionine on the HAMD (F=0.0093 and F<0.0001) and CGI (F=0.0038 and F<0.0001). BDI scores improved significantly for lower dose patients by ANOVA (F=0.0347) and from baseline to endpoint (t=4.2280, p=0.0242) for the higher dose. Mean termination hCg levels were 22.27umol/L for the 10g group and 13.2umol/L for the 5g group, where the upper reference limit is 15umol/L.

The data indicated that a lower dose of L-methionine in the combination treatment of L-methionine, betaine and folate maintained efficacy in the treatment of acute unipolar depression. Additionally, safety of the treatment was improved, with hCg levels within normal range with the lower dose.

### Example 5

Fourteen patients (9 women, 5 men) entered the trial and remained for 4.9±1.9 weeks, with nine completing the protocol and five dropping out after 2.8±1.8 weeks (1 nauseated in week 1; 2 lost to follow-up at weeks 1.5 and 4; 1 respiratory infection at wk 3; 1 dizzy at week 5). The study involved 20 hours of direct involvement for subjects to complete 10 Study Clinic Visits (one for screening, one for baseline, two in week 1, two in week 2, one each for weeks 3, 4, 5, 6). It lasted 6-8 weeks. Each Study Clinic Visit took about 2 hours. Treatment with L-methionine, betaine and folate was carried out for 6 weeks.

The study was entirely outpatient. Hospitalization is not part of the study. The subjects were 20 unipolar depressed patients, ages 18-64, with DSM-IV diagnosis of a Major Depressive Disorder (MDD) or Depressive Disorder NOS. They had a Hamilton Depression Rating Scale score of ≥ 18, and clinical indication for treatment of current depressive symptoms, without active substance abuse. Each subject signed an informed consent before entry to the study. Women of reproductive potential used an acceptable method of birth control, such as oral contraceptive, intrauterine device, diaphragm with contraceptive foam, condom with contraceptive foam, or abstinence. Subjects did not have a history of medical illness that would indicate a secondary mood disorder. Since the potential adverse events of most concern for the study would potentially arise from drug interactions or in individuals with specific risk factors, subjects were carefully screened for and excluded by medical conditions or concomitant medications or herbal agents that would have contraindicated a trial of the study regimen.

### Concomitant Medications and "Wash-Out"

The regimen of L-methionine, betaine and folate was the only psychotropic treatment permitted during the study, except for benzodiazepines. Lorazepam, up to a maximum of 2 mg/day is available (pm) for anxiety or insomnia during the study. Patients taking benzodiazepines prior to the study, except alprazolam, were not allowed to continue these medications throughout the study at the same dose as at study entry, unless the benzodiazepine was determined to be producing excessive adverse effects. No other psychotropic medications were permitted throughout the study. All subjects had received no psychotropic medication, except benzodiazepines, for a least one week prior to starting the study regimen. Potential subjects who had recently received MAO inhibitors or fluoxetine entered the study, but could not have received these medications for at least two weeks prior to receiving the study regimen.

There was no "washout" of psychotropic medication only in the service of study eligibility. A potential subject was eligible for the study if already tapered off psychotropic medications at study entry for clinical reasons unrelated to the protocol. Additionally, a potential subject was eligible for the study if they were currently being tapered off their psychotropic medications, but only if their psychiatrist or primary care physician initiated the taper for clinical reasons unrelated to the protocol, and their psychiatrist or primary care physician collaborated with performance and monitoring of the taper. Thus, enrollment in the protocol was restricted to subjects who were psychotropic medication naïve and/or subjects who were already tapered off or currently were tapering off their psychotropic medications.

Potential subjects who were receiving psychotropic medications and were treated in routine clinical practice by clinicians directly involved in the study could have entered the study, but only if they followed procedures concerning medication "washout." Again, a directly referred potential subject could have entered the study only if deemed appropriate for the study and capable of providing informed consent by another clinician not directly involved in the study. Such potential subjects were not be subject to a "washout" of psychotropic medication *only* in the service of study eligibility. Thus, the appropriateness of tapering psychotropic medication in the potential subjects were judged by another clinician not directly involved in the study and documented explicitly by the other clinician on the Direct Referral Patient Form.

If subjects agreed, they were tapered off herbal or alternative medicine preparations or teas, using a one week taper and a one week "wash-out" period prior to treatment with the regimen of L-methionine, betaine and folate. To determine usage of herbal or alternative medicine agents, patients responses to the following questions were evaluated: (a) "Do you take any herbal, alternative medicine or nutritional supplements or teas ?"; (b) "do you use St. John's wort, Ginko biloba, Kava kava, SAMe, Omega-3-fatty acids, fish oil, flax seed oil, valerian root, tryptophan, 5-hydroxytryptophan, 5-HTP or melatonin ?".

Subjects taking SSRI's (fluoxetine, sertraline, paroxetine, fluvoxamine, d,1-citalopram, 1-citalopram), SNRI's (venlafaxine, duloxetine), chlorimipramine, monoamine oxidase (MAO) inhibitors (phenelzine, tranylcypromine, isocarboxazide, meclobomide), tryptophan and 5-hydroxytryptophan were specifically excluded from receiving the study regimen. Serotonin syndrome has been reported to be associated with concomitant SAM (i.m.) and chlorimipramine (Iruela et al 1993), thus the restrictions. Subjects receiving the MAO inhibitor phenelzine, as well as the antitubercular agent isoniazide, required specific exclusion due to possible vitamin B6 depletion by these agents. Vitamin B6 is a cofactor in the one-carbon cycle.

Pseudoephedrine, dextromethorphan and meperidine were specifically prohibited during the study, but a tyramine-restricted diet was not required. The potential psychotropic nature of dextromethorphan and meperidine was reason enough to preclude their use during this study; a similar argument can be made for pseudoephedrine.

Alcohol was also specifically prohibited during the study. Alcohol can inhibit methionine synthase (Barak et al., Alcohol, 26:65-67, 2002), interfering with the one-carbon cycle and could possibly compromise the efficacy of the study regimen. Caffeine use was limited to 12 oz. per day to attenuate the confound of caffeine-induced anxiety; nicotine use was not prohibited or restricted.

### The Treatment Trial

Treatment was for 6 weeks with fixed oral doses of 5 grams/day L-methionine, 10 grams/day betaine, plus 5 mg/day folate. Starting doses were fixed, but decreases in dose were permitted if continued adverse effects did lead to loss of the subject from the study. Specifically, L-methionine was reduced to 5 g/day beginning with the sixth patient entering the study due to increased homocysteine levels in 2 of the first 5 patients in the study; the betaine and folate doses remained the same throughout the study. Lorazepam, up to a maximum of 2 mg/day was available (pm) for anxiety or insomnia during the study. Patient taking benzodiazepines, except alprazolam, were allowed to continue these medications throughout the study at the same dose as at study entry, unless the benzodiazepine was determined to be producing excessive adverse effects.

Six week trials are commonly utilized in assessing psychiatric medications as many agents require at least two weeks to begin to yield improvements, and patients may often continue to improve up to six weeks into the treatment. Longer trials subject non-responders to possibly ineffective therapy and incur greater expense. Shorter trials risk missing slow responders and are more confounded by spontaneous improvement of mood.

Following successful completion of the six weeks, subject participants were given the opportunity to voluntarily participate in a 1-2 month follow-up. During this period, patients were monitored as they were tapered off L-methionine, at a rate of 0.5 grams (1 pill) every day. Patients stayed on the same doses of trimethylglycine and folate, which was provided free of charge. Patients were advised to discontinue L-methionine following the completion of the study, therefore the free follow-up allowed for the transition to be accompanied by psychiatric monitoring. During the follow-up period, patients were advised as to which companies provided the supplements, so if they continued the betaine and folic acid independently after the follow-up period

### Statistical Considerations and Outcome Measures

Open trials reporting efficacy for SAM in the treatment of depression have included as few as 9 patients (Lipinski et al 1984). In this study, repeated measures and paired comparisons were made. The repeated measures were on average correlated for individual patients, so that for a moderate-to-large effect size with measures having an average correlation of r = .50 to .80, with power = .80 and α = .05, the sample size needed was about 15 (Stevens 1996).

Clinical outcome measures included patient self ratings and clinician ratings. Baseline scores were obtained for all scales before initiation of treatment. All rating scales were also completed twice weekly for the first 2 weeks, then once per week for weeks 3, 4, 5 and 6. Thus, rating scores for all scales were available for 9 time points.

Patient self ratings included the Beck Depression Inventory (BDI), Feighnor-Layton Mania scale, Zung Anxiety Scale, and the Arizona Sexual Experiences Scale (ASEX). The Zung Anxiety Scale was used to enhance detection of anxiety symptoms. The Arizona Sexual Experience Scale is a self-report measure of sexual dysfunction.

Clinicians completed the Hamilton Depression Rating Scale (HAMD), Clinical Global Impression (CGI) scales for severity and improvement of depression, Brief Psychiatric Rating Scale (BPRS), Young Mania Rating Scale (YMRS), and the Global Assessment of Functioning (GAF). The BPRS was used to enhance detection of psychotic symptoms. The YMRS was used to enhance detection of manic symptoms. The CGI scales were used to enhance detection of clinical worsening (see Off-Study Criteria), as well as improvement upon completion of treatment. The GAF is a standard scale of functional impairment.

Adverse effects reported in the literature for L-methionine, betaine or folate, such as anxiety, dizziness, sedation, nausea and diarrhea, were generally elicited, directly elicited, and formally evaluated. Thus, adverse effects were elicited generally by the question "have you had any adverse effects from the medication." More systematic evaluation of adverse effects were obtained using a formal scale for adverse effects (enclosed) and by performing a review-of-systems at each Study Clinic Visit.

The time course of clinical effects were evaluated with the BDI and HAMD using repeated measure analysis of variance. Clinical response rates were examined with HAMD using paired t-tests, with each patient as their own control. Efficacy was also judged by identifying "responders" and using a Chi-square test to compare the actual number of responders to the null hypothesis that there were no responders. Sensitivity to response was assured by identifying responders as patients meeting at least one of the following criteria. Patients with 50 % or greater decrease in Hamilton depression score or with Hamilton depression scores less than 12 were judged responders. Additionally, a "much improved" (moderate) or "very much improved" (marked) CGI improvement rating for depression was also judged as a response. Inclusion Criteria was as follows:
a. Age 18-64.
b. DSM-IV diagnosis of a Major Depressive Disorder (MDD) or Depressive Disorder NOS.
c. Clinical indication for treatment of current depressive symptoms.
d. Hamilton Depression Score of ≥ 18.
e. Each subject must sign an informed consent before entry to the study.
f. Women of reproductive potential must use an acceptable method of birth control, such as oral contraceptive, intrauterine device, diaphragm with contraceptive foam, condom with contraceptive foam, or abstinence.

Exclusion Criteria was as follows:
a. Treatment with another psychotropic drug, except a benzodiazepine (but no alprazolam). Smokers or users of any tobacco product, or users of the nicotine patch or any nicotine replacement product, were not be excluded.
b. Patients receiving SSRI's (fluoxetine, sertraline, paroxetine, fluvoxamine, d,1-citalopram, 1-citalopram), SNRI's (venlafaxine, duloxetine), chlorimipramine, monoamine oxidase (MAO) inhibitors (phenelzine, tranylcypromine, isocarboxazide, meclobomide), meperidine (Demerol), isoniazidc,or Coumadin.
c. Intent to continue or initiate herbal preparations, alcohol use, tryptophan or 5-hydroxytryptophan, pseudoephedrinc or dextromethorphan during the study.
d. History of mania or hypomania.
e. History of bipolar illness in first-degree relatives.
f. History of psychosis.
g. Active suicidal ideation with plan and intent.
h. History of a significant suicide attempt.
i. Active homicidality.
j. History of serious assault on another person.
k. Pregnant, trying to become pregnant, or nursing.
l. History of medical illness that would indicate a secondary mood disorder.
m. History of seizures or significant head trauma (i.e. sequelae occurred).
n. Current substance abuse within the past 1 month.
o. Positive urine drug screen.
p. History of sulfa allergy.
q. History of medical illness that would contraindicate a trial of the combination treatment, such as homocystinuria, hypertension, diabetes, hypercholesterolemia, coronary artery disease, myocardial infarction, angina, previous abnormal heart stress test, stroke, peripheral vascular disease, deep vein thrombosis, claudication, cardiac valve replacement, or renal insufficiency.

Off-Study Criteria included:
1.) Development of acute medical contraindications to L-methionine, betaine and folate therapy as outlined in the patient selection criteria above.
2.) Lack of clinical Efficacy, evidenced by a CGI-I rating of "worse" or "much worse".
3.) Development of intolerable adverse drug reactions as described above.

### Initial Physical Examination and Laboratories

An initial physical examination was performed in the screening of all potential subjects. Clinical laboratories performed to screen all subjects included: blood homocysteine level, creatinine, liver function tests, B12 level, and free T4. All subjects had a urine drug screen (cocaine, opiates, amphetamine, tetrahydocannabinol) performed prior to study entry. In females, a urine β-Human Chorionic Gonadotropin test to exclude pregnancy was performed.

### (Figure 13: NARSAD Evaluation)

### Clinical Evaluation & Parameters Measured

Patients received ongoing psychiatric ratings as described above. Patients had vital signs monitored at least weekly. Blood homocysteine level, liver function tests and erythrocyte sedimentation rate (ESR) were obtained at the end of the study, as well as baseline (Figure 13). Blood homocysteine level and liver function tests were compared between baseline and study end to evaluate adverse effects on these measures. ESR was compared between baseline and study end as a proxy to evaluate the hypothesis that the study regimen increased cell membrane fluidity. Additional clinical laboratories were monitored as clinically indicated.

### L-Methionine, Betaine and Folate Dosing

L-methionine and folate are essential nutrients for humans, with a daily requirement of 2 grams for L-methionine and 400 mcg. for folate (Lehninger Principles of Biochemistry, Worth, New York, 1982). Additionally, about 0.5 to 2 grams per day of betaine is contained in an average diet (Olthof et al., J. Nutr., 133:4135-4138, 2003).

Folate was dosed at 5 mg orally (PO) daily to prevent depletion by the exogenous L-methionine and the augmented one-carbon cycle. Studies examining exacerbation of schizophrenia used 10-20 grams/day of L-methionine and betaine (Baldessarini, Int. Rev. Neurobiol., 18:41-67, 1975). Studies of oral betaine treatment of homocysteinuria have used 10-20 grams/day (Wendel et al., Eur. J. Pediatr., 142:147-150, 1984), 2-9 grams/day (Ogier de Baulny et al., Eur. J. Pediatr., 157(suppl 2):S77-83, 1998), and 6 grams/day (Wilcken et al., N. Eng. J. Med., 309:448-453, 1983). The methionine loading test for hyperhomocysteinemia is generally performed with 100 mg/kg, or about 7 grams/day for a 70 kg person. A study of oral betaine in Alzheimer's disease used 3 mg bid (Knopman et al., Alzheimer Dis. Assoc. Disord., 15:162-165,2001). Studies of betaine benefits in hemodialysis patients used 4 grams/day. Finally, the DRL-72 second operant schedule studies in rats that found L-methionine and betaine augmented the antidepressant effects of a β-adreneric agonist used 90 mg/kg, which would be 6.3 grams for a 70 kg. person.

The study used oral doses of 5 grams/day of L-methionine and 10 grams/day of betaine. Folate was dosed at 5 mg PO once daily. Thus, 2.5 times the daily requirement of methionine, 13 times the daily requirement of folate, and 5-10 times the average daily intake of betaine was administered. Starting doses L-methionine and betaine were fixed, but decreases in dose were permitted if continued adverse effects would lead to loss of the subject from the study.

Some of the adverse effects of L-methionine and betaine were monitored by twice daily (BID) dosing; blood pressure was also monitored at least weekly. Additionally, as methionine is a sulfur-containing molecule, and although not strictly a "sulfa" compound, subjects with sulfa allergy were excluded. The risk of psychosis and mania from L-methionine and betaine was reduced by screening out patients with a history of psychosis or mania, such as psychotic depression, schizophrenia, bipolar or schizoaffective disorder. Additionally, symptoms of psychosis and mania were evaluated at 9 time points over the course of the study, including use of the Brief Psychiatric Rating Scale (BPRS) and Young Mania Rating Scale (YMRS). Note that most studies finding worsening of schizophrenia with methionine loading had co-administered an monoamine oxidase (MAO) inhibitor to enhance the potential production of postulated transmethylated psychotogens.

To avoid clinical risk from any hyperhomocysteinemia that could be produced by L-methionine, patients were screened out with homocystinuria, renal failure, or significant cardiovascular risk factors, and by co-administering betaine and folate. Patients with cardiac valve replacement or receiving Coumadin were be excluded from the study. Also excludes, were patients with coronary artery disease or significant risk factors for coronary artery disease (hypertension, diabetes, hypercholesterolemia, coronary artery disease, myocardial infarction, angina, previous abnormal heart stress test, stroke, peripheral vascular disease, deep vein thrombosis, claudication). Finally, renal failure is associated with hyperhomocysteinemia, so patients with renal insuffiency were excluded from the study.

The risk of clinical worsening during the study is reduced by evaluating patients for depression, anxiety, psychosis, mania and other adverse effects at 9 time points over the course of the study, and have as "Off-Study" criteria a CGI-I rating of "worse" or "much worse" at any of these 9 time points. Note that a potential subject were eligible for the study if being tapered off their psychotropic medications. Such subjects were at an increased risk of worsening of symptoms during the taper. These subjects only entered the study if their psychiatrist or primary care physician initiated the taper for clinical reasons unrelated to the protocol, and their psychiatrist or primary care physician collaborated with performance and monitoring of the taper. The study was entirely outpatient. Hospitalization was not part of the study.

### Efficacy in Depression

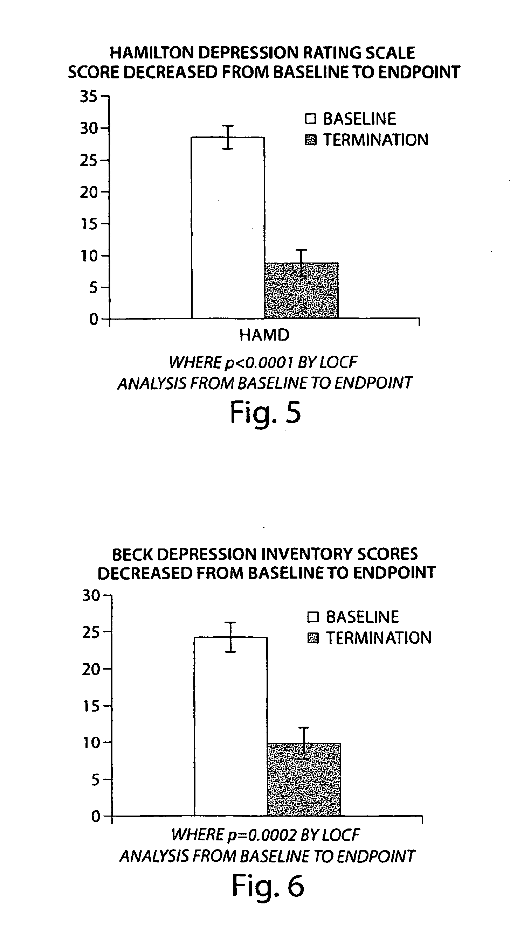

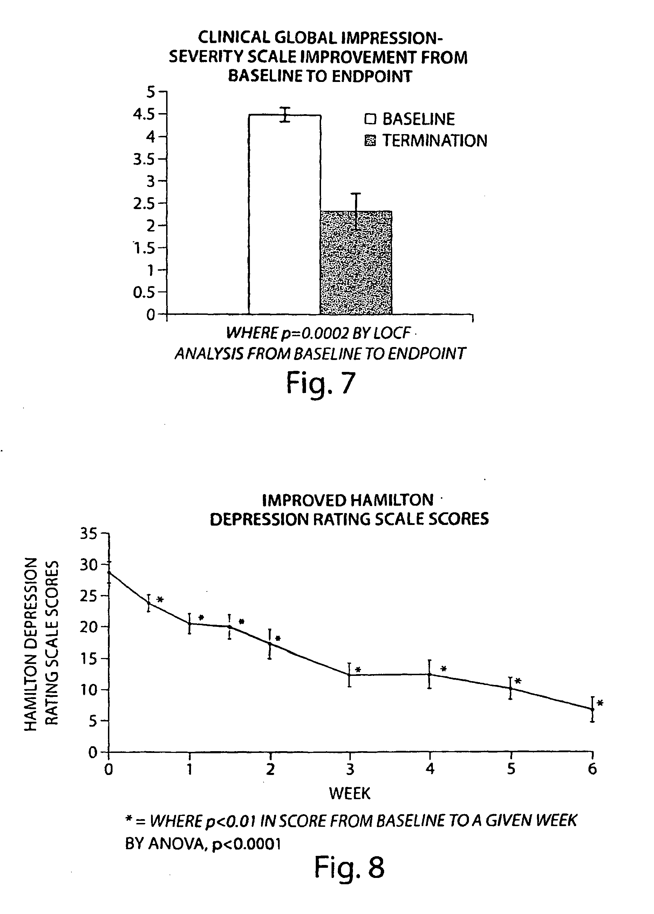

After treatment with the L-methionine, betaine, and folate (MBF) mixture containing a daily dose of L-methionine of 10 grams in five and of 5 grams in nine cases, depressive symptom scores on the HDRS-28 and other scales were obtained in all 14 patients at baseline and 13 at a later end-point averaging 4.9 weeks, showed improvements from (28.5±5.80 to 9.00±5.94) or by 68.4% (LOCF: t = 9.34, p<0.0001) (Figure 5). HDRS-17 also showed similar improvements (17.8±4.28 to 5.77±4.34, or 67.6%; t = 9.97, p<0.0001). Beck Depression Inventory (BDI) scores also indicated major improvement (24.7±6.84 to 10.3±7.03 [58.3%]; t = 6.00, p=0.0001(see Figure 6), as did CGI-severity scores (4.46±1.32 to 2.31±0.52 [48.2%], t = 6.06, p=0.0001 (see Figure 7)). Post-hoc analysis suggested that improvement occurred as early as the first week (see Figure 8).

### Effective for Atypical as well as Typical Depression

Of the 14 patients, 8 were considered to have typical, and 6 atypical depressive symptoms. HDRS-28 scores (which include both types of symptoms) improved similarly in both subgroups, from 28.4±5.78 to 10.9± 2.29 (61.6%) among patients with typical depression vs. from 28.6 ± 6.50 to 6.00± 3.81 (79.0%) in those with atypical symptoms (both t=2.20, both p=0.0002). Similarly, both subgroups showed improved BPRS ratings (from 35.5±5.37 to 24.8±4.89 [30.1%] and 31.6±4.04 to 22.2±2.17 [29.7%], respectively; both t=1.87, both p<0.005) as well as CGI (from 4.50±0.53 to 2.75±1.39 [38.9%] and 4.40±0.55 to 1.60±0.89 [63.6%], respectively; both t=2.16, both p=0.0003) and BDI scores (from 25.4±8.48 to 11.5±7.05 [54.7%] and 23.6 ± 3.43 to 8.40±7.33 [64.4%], respectively; both t=3.17, both p=0.03).

### Efficacy for Anxiety

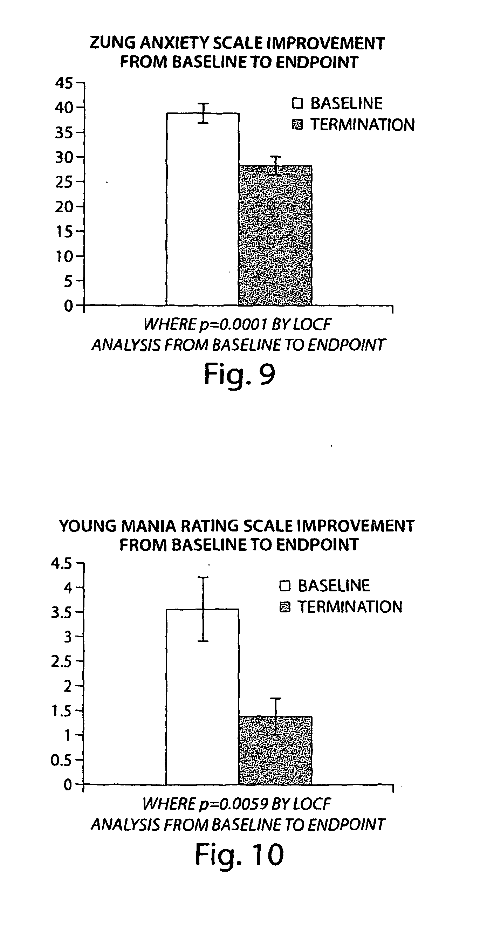

Zung Anxiety scale scores (n=13) also decreased from baseline (38.4±6.75) to endpoint (28.2±6.01), or by 26.6% (t = 6.23, p<0.0001). (see Figure 9)

### Risk of Mania or Psychosis

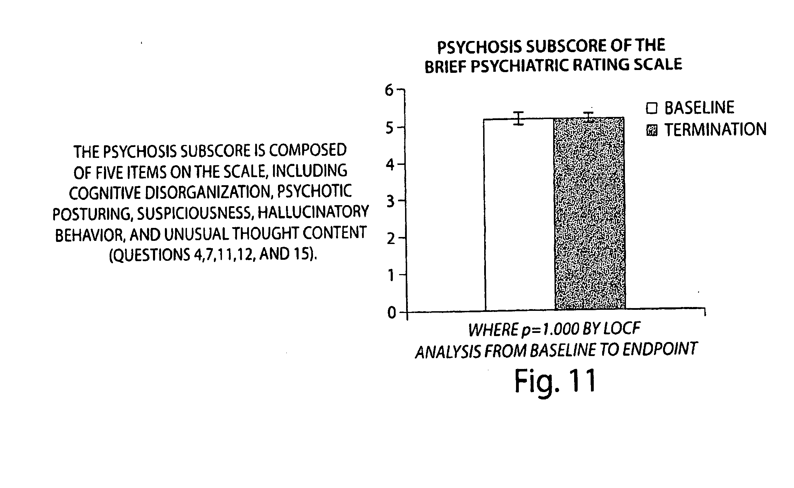

YMRS mania-scale ratings did not increase, but instead showed an average decrease of 60.4% between baseline (3.31±2.39) and endpoint (1.31±1.25) in LOCF analysis, where (t = 3.28, p=0.007) (Figure 10). BPRS psychosis symptom ratings were unchanged (from 5.15 ± 0.55 to 5.15±0.38; t= 0.00, p=1.00). (see Figure 11).

### Effects of a Lower Dose of Methionine

At only 5 g/day of L-methionine, 9 patients showed major improvements in several ratings. HDRS-28 scores (t =2.73, p<0.0001), CGI (t = 2.98, p<0.0001), and BDI scores (t =1.70, p=0.009) all improved significantly between intake and endpoint.

### Adverse Effects

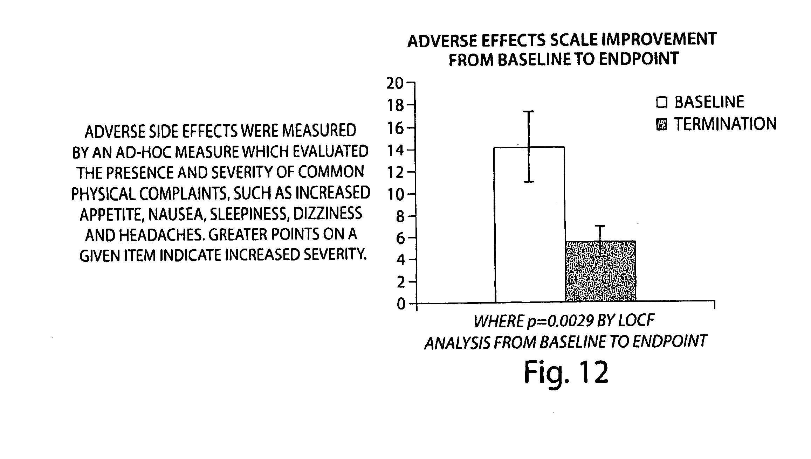

NIMH Adverse Effects scale total scores also did not increase, but decreased by 61.9% (from 13.5±10.3 to 5.15±4.89; t =4.02, p=0.002) (see Figure 12). Serum homocysteine concentrations at endpoint were 22.3±6.51 µmol/L at a daily dose of L-methionine of 10 g and 12.2±5.10 µmol/L at 5 g, compared to an upper-limit of normal of 15.0 µmol/L (van Guldener et al., J. Int. Med., 245:175-183, 1999). There were no indications of altered hepatic functioning. Body-weight remained stable (198±65 at both intake and endpoint), as did heart rate (71±6 vs. 73±10; t= 0.95, p=0.37), systolic (125±10) vs.122±14 mm Hg; t = 0.78, p=0.46), and diastolic blood pressure (78.1±7.1 vs. 76.7±10; t = 0.62, p=0.55). Finally, total ASEX scores were very similar at baseline (10.8±2.96) and endpoint (10.3±4.10; t = 0.45, p=0.67).

The study in 14 patients indicates that treatment with the combination of L-methionine, betaine and folate is effective for depression and anxiety, and has a low incidence of adverse effects. Measures of depression and anxiety are both significantly improved. Patients with atypical depression (hypersomnic, hyperphagic) as well as typical depression (hyposomnic, hypophagic) were improved. The treatment was well tolerated, except for increased homocysteine levels in some patients. The increased homocysteine levels in patients receiving the regimen with 10 grams/day of L-methionine (mean homocysteine level was> 15 µmol/L) led to a decision to reduce the L-methionine dose to 5 grams/day, with no loss of efficacy and a mean homocysteine level in the normal range (Dunn et al 2007b).

### Power Analysis

Utilizing the means and sigma (standard deviation) for efficacy in depression for the HAMD-28 from our previous open study, with the assumptions that:
1. µ 1=HAMD-28 for placebo at endpoint of controlled study = baseline HAMD-28 for open study = 29,
2. µ 2=HAMD-28 active treatment at endpoint of controlled study = endpoint HAMD-28 for open study = 9
3. σ for HAMD-28 for controlled study = sigma for HAMD-28 at endpoint of open study = 10
4. α=0.01
5. power=0.90,
   then the sample size for each group that is needed to detect Efficacy of the active treatment in depression is n=30.

Utilizing the means and σ for efficacy in anxiety from the ZAS (Zung Anxiety Scale) for which using data from the study in example 5, rather than the HAM-A which was the primary anxiety outcome measure for the controlled study, with the assumptions that:
1. µ1=ZAS for placebo at endpoint of controlled study = baseline ZAS for open study = 38
2. µ2=ZAS for active treatment at endpoint of controlled study = endpoint ZAS for open study = 28,
3. σ for ZAS for controlled study =sigma at endpoint of open study = 2,
4. α =0.01
5. power=0.90,
then the sample size for each group that is needed to detect efficacy of the active treatment in anxiety is n=31.

### Example 6

An open-label, non-randomized, prospective 6-week study of 20 outpatients was conducted to examine the efficacy and tolerability of a combination of L-methionine, betaine and folate in treating unipolar depression. Patients attended twice weekly visits for two weeks, then weekly visits thereafter. At each visit, psychiatric and physical outcomes were measured using the Hamilton Depression Rating Scale - 28 item (HDRS-28; Reimherr et al., Am. J. Psychiatry, 155 (9), 1247-1253, 1998), Young Mania Rating Scale (YMRS), Brief Psychiatric Rating Scale (BPRS; Overall et al., Psychol. Rep., 10, 799-812, 1962), Clinical Global Impression Scale (CGI; Guy et al., Assessment manual for psychopharmacology. DHEW publication (ADM). 76-338, US Department of Health, Education, and Welfare, Washington (DC), 1976), and the Global Assessment of Functioning (GAF). Self reports included the Beck Depression Inventory (BDI; Beck et al., Archi. Gen. Psychiatry, 4, 561-571, 1961), Zung Anxiety Scale (ZAS; Zung, Psychosomatics, 12, 371-379, 1971), Arizona Sexual Experiences Scale (ASEX) (McGahuey et al., J. Sex Marital Ther., 26, 25-40, 2000), and the NIMH Adverse Effects Scale (AES). The AES is a self-assessment scale which lists several common medication side effects and allows patients to report their presence and severity. Additionally, at baseline and endpoint, laboratory tests were performed and vitals were obtained, including liver function tests, erythrocyte sedimentation rate (ESR), homocysteine (hCy) levels, free thyroxine levels, vitamin B12 levels, urine drug screen, urine pregnancy test, pulse, blood pressure, and weight.

Dosages for this study were initially fixed, with oral doses of 10 grams/day L-methionine, 10 grams/day betaine, plus 5 mg/day folate. However, after the initial five patients, L-methionine dosage was reduced to 5 grams/day, due to increased hCy levels at termination in some patients. For individual patients, decreases in dose were permitted if adverse effects were significant. Lorazepam, up to a maximum of 2 mg/day was available as needed for anxiety or insomnia during the study.

The L-methionine was assayed at 99% - 101% purity for 500mg capsules with trace contaminants at less than 200ppm. Inert ingredients were magnesium stearate and gelatin capsule. The betaine was assayed at 101.09% purity for 1000mg tablets with trace contaminants at less than 0.07ppm. Inert ingredients included dicalcium phosphate, microcrystalline cellulose, croscarmellose sodium, stearic acid, silica, magnesium stearate, cellulose, titanium dioxide, and riboflavin. The folate was assayed at 100% yield for 5mg tablets, and inert ingredients included microcrystalline cellulose and gelatin.

All patients met DSM-IV diagnostic criteria for Major Depressive Disorder (MDD) or Depressive Disorder Not Otherwise Specified (NOS). Additionally, patients met mood scale criteria, which included a HDRS-28 rating ≥ 18, limiting the study population to those with moderate to severe depression. Patients were aged 18-64 and were required to provide written informed consent for study participation. Women of reproductive potential were required to use an acceptable method of birth control and have a negative urine pregnancy test. Concomitant psychotropic medications and psychoactive herbal preparations were not permitted, with the exception of the aforementioned lorazepam. Alcohol and caffeine use were limited during study participation. Exclusion criteria included history of mania or hypomania, bipolar illness in any first degree relative, history of psychosis, a past significant suicide attempt, active suicidal ideation with plan and intent, history of serious assault on another person, active homicidality, and active substance abuse. Medical exclusions included history of a medical illness which would indicate a secondary mood disorder, history of seizures or significant head trauma with sequelae, sulfa allergy, and a history of any medical illness which would contraindicate a trial of the combination treatment, including elevated hCy level homocystinuria, hypertension, diabetes, hypercholesterolemia, coronary artery disease, myocardial infarction, angina, previous abnormal heart stress test, stroke, peripheral vascular disease, deep vein thrombosis, claudication, cardiac valve replacement, and renal insufficiency. Baseline creatinine was taken to confirm no renal insufficiency. Free thyroxine and vitamin B12 levels were taken to confirm that mood symptoms were not secondary to medical illness.

Outcomes were analyzed predominantly by intent-to-treat and last observation carried forward (LOCF) methods, but also by repeated measure ANOVAs with the Tukey post-hoc test of significance at distinct time points. The primary outcome measure was change in HDRS-28 scores. Response was defined as ≥50% reduction in HDRS-28 score. Secondary outcomes were BDI, CGI-severity, and ZAS scores. Adverse effects were monitored using AES, BPRS, YMRS and ASEX scores, as well as hCy levels, ESR, liver function panels, blood pressure, pulse and weight. Mania induction was monitored using the YMRS. Induction of psychosis was monitored using a subscale of the BPRS, which included items 4 (disorganized thought), 7 (psychotic posturing), 11 (suspiciousness), 12 (hallucinations), and 15 (unusual thought content).

### Results

Baseline measures were completed by twenty depressed unipolar outpatients. Ages ranged from 23 to 64 years, with a mean age of 45.5 (SD = 12.06). The sample included 8 men and 12 women. Sixteen of the 20 patients were Caucasian, 3 were Asian, and 1 was African American. Six patients (30%) had a history of substance abuse. All demographic characteristics of the sample are summarized in the table below.

### Demographic Information

| Patient characteristics | |
|---|---|
| Age: Mean ± SD (range) (yrs) | 45.4 (SD =12.06) |
| Gender (male: female) | 8: 12 |
| Race (Caucasion: Asian: African American) | 16: 3: 1 |
| Hx of Substance Abuse (yes/no) | 6: 14 |
| HDRS-28 Responder (%) | 70% (14 of 20) |
| Completers (N) | 15 |

Fifteen patients (75%) completed the full six weeks of treatment. The mean duration of treatment for the entire sample was 5.2 weeks (SD = 1.64). One patient ended treatment after the baseline visit and another ended the study prior to week 2 of the study. Additionally 3 patients ended treatment between weeks 3 and 5. These patients terminated early for reasons including side effects (n=2), unrelated upper respiratory infection (n=1), patient choice (n=1), and loss to follow-up (n=1). Side effects reported by early terminators included dizziness (n=2), nausea (n=1), lethargy (n=1), and flank pain not associated with hematuria, urinary tract infection or abnormal renal function (n=1). No patients left the study due to a worsening of mood or were hospitalized during or as a result of study participation.

Treatment was begun with fixed doses of MBF. One patient took a reduced dose of 1.5grams/day L-methionine, 3 grams/day betaine, and 5mg/day folate for one week due to agitation during the study. This patient was not a responder. Two patients were given lorazepam during the study. The first received lorazepam for one week in order to treat anxiety and the other received lorazepam for two weeks for the treatment of insomnia. All other patients were administered MBF monotherapy at a fixed dose for 6 weeks. As mentioned earlier, dosage of L-methionine was 10 grams/day for the first 5 patients in the study. This dose was reduced to 5 grams/day for the rest of the patients due to increased hCy levels at termination in some patients

Seventy percent (n = 14) of the 20 patients, and 80% (n=12) of the 15 completers were deemed responders, as indicated by a ≥ 50% reduction from baseline HDRS-28 scores. A Chisquare analysis was conducted to test the significance of this outcome. This response rate was found to be statistically significant when a 30% chance of placebo response was assumed (χ² (1, *N* = 20) = 10.67, *p* < .01) or when a 40% chance of placebo response was assumed (χ² (1, *N* = 20) = 4.5, *p* < .05). Furthermore, improvement in HDRS-28 scores was seen in an LOCF analysis of the baseline HDRS-28 scores in comparison to the HDRS-28 scores at termination (including those who terminated early). Mean baseline HDRS-28 score was 28.84 (SD = 6.66) and mean termination score was 10.05 (SD = 6.3), and 50% of the sample had a score lower than 10 on the HDRS-28 when ending the study. This change is statistically significant as indicated by the LOCF analysis (*p* < 0.001). Furthermore, calculation of the effect size between the mean HDRS-28 scores at baseline and endpoint resulted in Cohen's *d* = 2.87, decreasing the possibility that type I errors were present and confirming the significance of our findings.

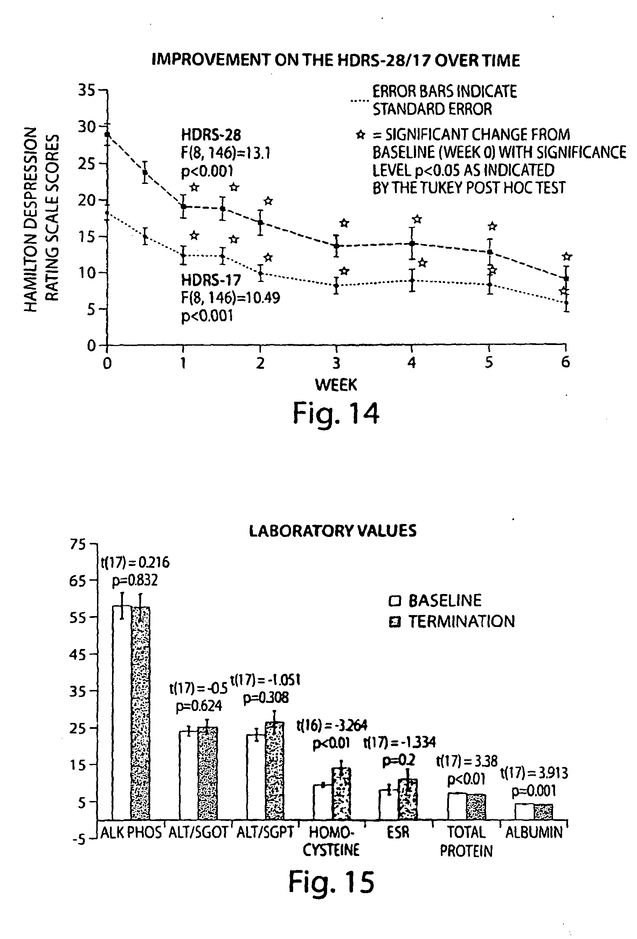

The time-course of improvement was seen in the analysis of variance of HDRS-28 scores. The improvement in HDRS-28 scores was rapid and persistent, as seen in a week by week ANOVA for change over time in total HDRS-28 scores. In fact, a significant improvement in HDRS-28 scores from baseline was seen at week 1 of the study (*F* (8,146) = 13.1, *p* < 0.001; Figure 14, and the table below).

### Treatment Characteristics

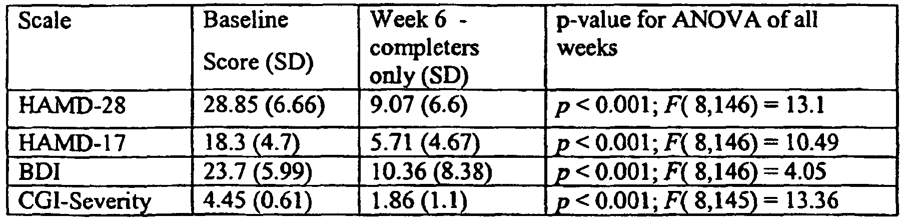

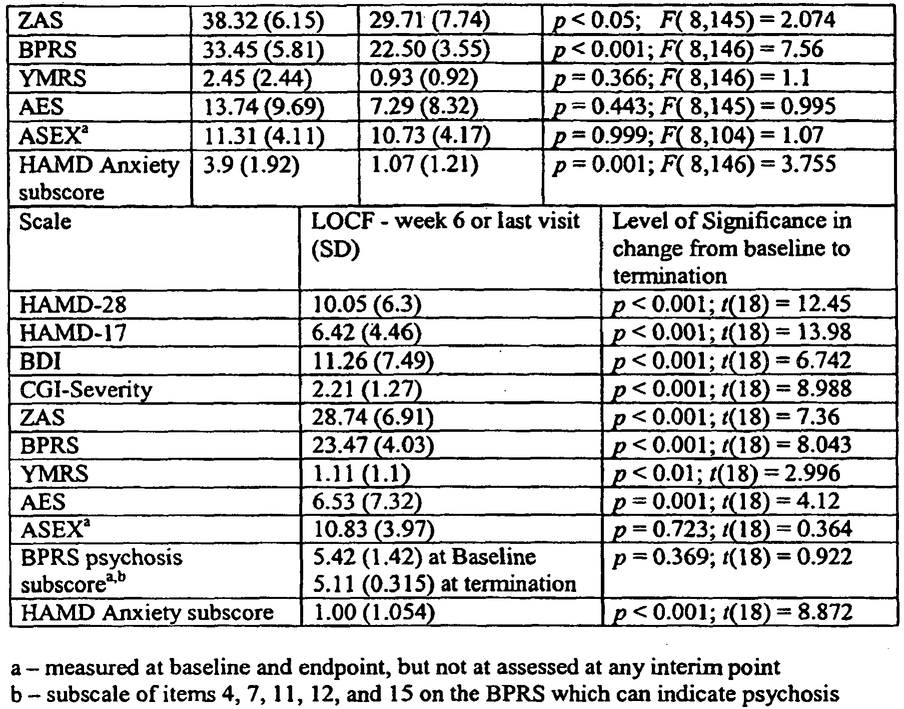

By week 2, the mean HDRS-28 score of all patients was below 18, the necessary minimum score for study entry.

These findings were further supported by an analysis of the Hamilton Depression Rating Scale-17 (HDRS-17 and the demographic information table above), which showed an improvement in scores from baseline to endpoint (F(8, 146)= 10.49, p < 0.0001). The HDRS-17 is composed of the first 17 items of the HDRS-28 and is commonly used to assess antidepressant efficacy (Williams et al., Eur. Arch. Psychiatry Clin. Neurosci., 251, Suppl 2: II 6-12, 2001). It excludes the last 11 items of the HDRS-28 that measure some atypical symptoms of depression, such as increased sleep, appetite, and weight. We divided the sample into two groups of patients. Those with higher scores on the HDRS-28 items that measure decreased sleep, appetite, and weight were considered to have typical depression, while those with higher scores on items measuring increased sleep, appetite and weight were considered to have atypical depression. Ten patients fit into the typical depression group and 10 fit into the atypical depression group. No differences were found between the group of patients showing typical depressive symptoms at baseline and those showing atypical depression symptoms at baseline, in terms of the number of responders (p=0.36) or level of improvement in depression (p=0.61). Both groups showed significant improvement in mean total HDRS-28 scores from baseline to endpoint, as seen both in an LOCF analysis (*p* < 0.001). An ANOVA also showed significant improvement over time for the group with typical depression (*F*(8, 72)= 6.842, p < 0.0001) as well as for the atypically depressed group (*F*(8, 65) = 5.638, p < 0.0001).

An ANOVA conducted on each of the 28 items in the HDRS-28 for the entire sample showed a significant decrease in scores of items measuring depressed mood (*F*(8,146) = 9.57, *p*<0.001), feelings of guilt (*F*(8,146)= 5.11,*p*<0.001), middle insomnia (*F*(8,146)= 3.69, *p*<0.001), loss of interest and decreased participation in work and activities (*F*(8,146) = 16.37, *p*<0.001), psychomotor retardation (*F*(8,146) = 2.54, p<0.05), agitation (*F*(8,146) = 2.55, *p*<0.05), anxiety (*F*(8,146) = 2.7, *p*<0.01), somatic anxiety symptoms (*F*(8,146) = 2.14, *p*<0.05), diurnal variation (*F*(8,146) = 2.64, *p*=0.01), fatigue (*F*(8,146) = 5.71, *p*<0.001), and social withdrawal (*F*(8,146) = 4.99, *p*<0.001). Note that this analysis showed an improvement in the cardinal symptoms of depression, depressed mood and anhedonia, as represented by the items measuring depressed mood, decreased interest and participation in work and activities, and social withdrawal. The findings were also confirmed by an analysis of the CGI-severity scores (See, demographic information table above). Finally, the clinician-rated measures were confirmed by patient reports of significantly improved depressive symptoms as indicated by ANOVA analysis of the self-rated BDI (*F* (8,146) =4.05, *p* < 0.001).

An additional finding was an improvement in anxiety symptoms. An ANOVA conducted on the ZAS scores showed a significant improvement in subjective anxiety symptoms as well (*F*(8,145) = 2.07, *p*<0.05). In fact, in its assessment of depression severity, the HDRS-28 also includes items relating specifically to anxiety symptoms. Items 9,10, and 11, were extracted from the HDRS-28 and constructed an anxiety sub-score comprised of the sum of these three items. An ANOVA of the HDRS-28 (*F*(8,146) = 3.755, *p*=0.001), as well as a paired t-test using the LOCF method on the data (p < 0.001), showed an improvement on this sub-score, indicating that the overall anxiety levels alleviated in this sample over the time of the study.

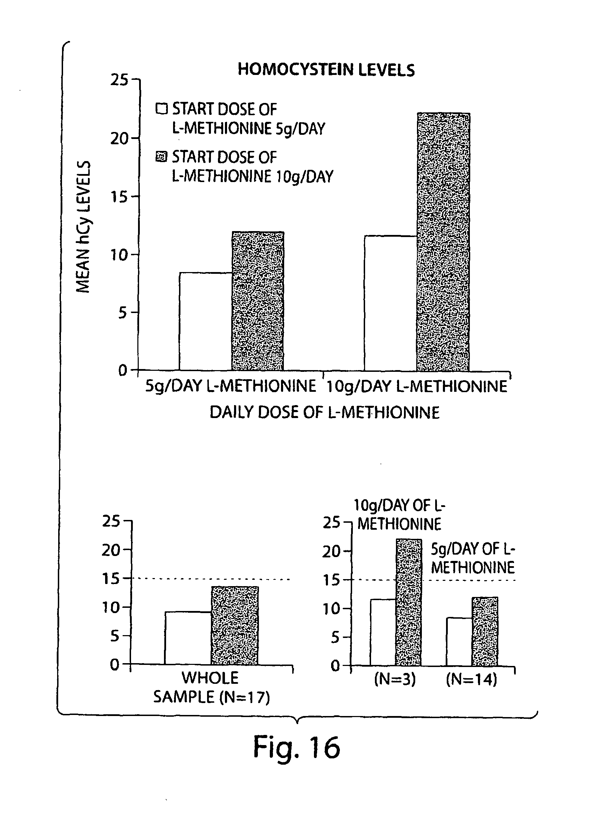

Despite observations of elevation in hCy levels in some patients, the mean termination hCy value for the entire sample was 13.87 ±1.79, which is not above the healthy range (Figure 16). The reference range for hCy values used for this study was 0-15 umol/L (Refsum et at, 2004). Three of the 5 patients who were started on 10g/day of L-methionine completed the study, and the mean termination hCy level for these 3 patients was 22.27 ± 6.5. Fourteen of the 15 patients in the 5g/day L-methionine dose group came in for their termination labs, and the mean termination hCy levels for this group was 12.07 ± 1.41. Mean endpoint hCy levels for the 5g/day methionine group were significantly lower than the 10g/day group (t(15) = 2.5, *p* = 0.025). A total of 7 patients had elevated homocysteine levels, which included 2 of 3 (67%) in the 10g/day L-methionine group and 5 of 14 (36%) in the 5g/day L-methionine group. Mean hCy increase in the 10g/day group of patients was 9.36 ± 9.71. For those patients on a reduced dose, mean hCy increase was 3.54 (SD = 4.48). There were no significant differences in HDRS-28 mean scores between the two groups that received different doses of L-methionine during any of the measurement points in the study.

No scale for adverse effects worsened nor was any mean laboratory value elevated outside of normal range during treatment (See, table regarding treatment characteristics above, the table below and Figure 15).

### Laboratory values

| | Baseline Mean (SD) | Termination Mean (SD) | Reference Range | p-value |
|---|---|---|---|---|
| Homocysteine | 9.3 (2.62) | 13.87 (7.39) | 0-15 | 0.005 |
| ESR | 7.94 (5.48) | 10.72 (11.636) | 0-15 | 0.200 |
| Total Protein | 7.05 (0.415) | 6.74 (0.287) | 5.9-7.5 | 0.005 |
| Albumin | 4.24 (0.24) | 3.98 (0.312) | 3.4-4.8 | 0.001 |
| Total Bilirubin | 0.744 (0.243) | 0.689 (0.196) | 0.2-1.1 | 0.35 |
| Direct Bilirubin | 0.112 (0.048) | 0.082 (0.0529) | 0-0.2 | 0.056 |
| Alkaline Phosphatase | 58.00 (15.703) | 57.5 (15.561) | 25-106 | 0.832 |
| Aspartate Aminotransferase (AST/SGOT) | 23.94 (5.286) | 24.00 (8.153) | 8-34 | 0.624 |
| Alanine Aminotransferase (ALT/SGPT | 22.778 (6.77) | 26.22 (13.41) | 7-35 | 0.308 |
| Weight | 173.33 (61.65) | 173.67 (62.57) | | 0.825 |
| Systolic Blood Pressure | 121.33 (10.84) | 118.83 (13.49) | | 0.451 |
| diastolic Blood Pressure | 76.67 (6.71) | 75.83 (9.25) | | 0.674 |
| Pulse | 71.58 (5.55) | 73.25 (9.85) | | 0.533 |

Neither induction of mania nor psychosis was seen during treatment, as observed through ANOVA of YMRS and BPRS psychosis subscores. Total BPRS scores improved significantly during treatment as well, as determined by an ANOVA (*F* (8,146) = 7.56, *p* < 0.001). There was no increase in ASEX or AES scores, nor was there an change in weight, pulse, systolic blood pressure or diastolic blood pressure. Mean laboratory values were within normal range at both baseline and endpoint of the study.

### Reduction in Symptoms of Depression

The primary and most notable outcome of this study was the fact that 70% of the patients in the sample responded to the treatment, as indicated by the HDRS-28. This finding was supported using both a t-test with the LOCF method and an ANOVA. Furthermore, this finding was supported by the analysis of the Beck Depression Inventory (BDI) scores over time, which also showed a significant improvement in mood of patients in the sample over the six weeks of the trial. The similar results between the HDRS-28, a clinician rated scale, and the Beck Depression Inventory (BDI), a patient rated scale, further validates our findings, as it shows that both subjective and objective depressive symptoms improved in this sample during the study.

Finally, the improvement in HDRS-28 items measuring typical and atypical depressive symptoms in this sample indicated that the L-methionine, betaine, and folate (MBF) combination may be successful in both types of depression.

Another finding of this study was the overall improvement in anxiety symptoms that was indicated by the Zung Anxiety Scale (ZAS). Symptoms of anxiety and depression often overlap, and patients treated for depression are often in need of treatment for anxiety symptoms as well. Therefore, the finding that symptoms of anxiety were alleviated in the current sample over the six weeks of the study is significant. These findings indicate that the MBF combination is useful to patients with anxiety disorders.

An important consideration when treating patients with MBF is the risk of increased hCy levels caused by the methionine. The findings showed that there was an overall increase in hCy levels, both in the 5g/day methionine group and in the 10g/day methionine group, however, mean termination hCy levels in the 5g/day group was significantly lower than the 10g/day group, and mean termination hCy levels in the 5g/day group were within the healthy range. The normal mean hCy levels at the end of the study for the 5g/day group may be due to the decreased daily dose of methionine.

The increase in hCy levels indicates that the protocol indeed augments the one-carbon cycle and likely increasing SAM. Mechanistically, SAM is involved in the synthesis of serotonin, dopamine and norepinephrine, thereby leading to improved depressive symptoms in the patients of this study. Consistent with this interpretation, methionine has been found to improve Parkinson's disease, suggesting that it enhances dopaminergic neurotransmission (Di Rocco et al., Movement Disorders, 15:1225-1229, 2000).

The data indicate that the treatment described herein is useful as an antidepressant treatment in unipolar depression.

### Example 7

Following the completion of a six week trial, in which patients were treated for depression with a combination of L-methionine, trimethylglycine (a betaine) and folate, participants were given the opportunity to voluntarily participate in a follow-up extension trial. During this period, patients were monitored for 10 days as they were tapered off L-methionine at a rate of 0.5 grams (1 pill) every day, where the starting dose was 5g/day. Patients remained on 10g/day of trimethylglycine and 5mg/day of folate and were assessed again for depression 2 weeks after the L-methionine was discontinued. Patients remained on these doses of trimethylglycine and folate throughout the follow-up period. Of the 20 patients in the sample, 3 patients volunteered to participate in this follow-up trial, and the mean duration in the follow-up trial for these patients was 3.33 weeks (SD = 1.16). An LOCF analysis comparing mean depression scores on the HDRS-28 at week 6 of the first trial (M = 8.33, SD = 9.7) and the mean depression scores on the HDRS-28 at end of participation in the extension period (M = 2.33, SD = 4.01) showed that depression remained improved when patients were on only the trimethylglycine and folate (t(2) = 1.73, p = 0.225). Furthermore, when compared with the baseline mean depression scores during the first trial period for these 3 patients (M = 22.67, SD = 4.04), the mean HDRS-28 scores were still significantly lower at termination of the extension period (t(2) = 4.6, p < 0.05). To conclude, the trimethylglycine and folate regimen alone was effective in maintaining the low depression scores of these patients.

## Claims

1. A therapeutic formulation comprising betaine, L-methionine and folate or folic acid in an amount that augments the endogenous synthesis of S-adenosylmethionine (SAM) produced in the one-carbon cycle for use in treating unipolar depression, wherein folate is the anion form of folic acid.

2. Use of betaine, L-methionine and folate or folic acid in the manufacture of a medicament for treating unipolar depression in a patient in need thereof, wherein the treating comprises administration to the patient of a therapeutic formulation comprising betaine and folate in an amount that augments the endogenous synthesis of SAM produced in the one-carbon cycle, wherein folate is the anion form of folic acid.

3. The formulation for use according to claim 1 or the use of claim 2, wherein an homocysteine increase in levels in the patient does not exceed 10 µmoles per liter compared to a pre-treatment level.

4. The formulation for use according to claim 1 or the use of claim 2, wherein the amount of SAM produced is between 100-180% times the normal amount of SAM produced in the patient prior to said administering, preferably 140% times the normal amount of SAM produced in the patient prior to said administering.

5. The formulation for use according to claim 1 or the use of claim 2, wherein:
(i) the dosage comprises between 1-6 times the daily requirement of L-methionine of the patient;
(ii) the dosage comprises 1.5-4 times the daily requirement of L-methionine of the patient;
(iii) the dosage comprises 2.5 times the daily requirement of L-methionine of the patient;
(iv) the dosage comprises between 1-20 times the average daily intake of betaine of the patient, preferably between 2.5-15 times the average daily intake of betaine of the patient, and more preferably between 5-10 times the average daily intake of betaine of the patient;
(v) the dosage comprises between 1-20 times the daily requirement of folate of the patient, preferably 10-18 times the daily requirement of folate of the patient, and more preferably 13 times the daily requirement of folate of the patient;
(vi) the dosage comprises between 1-10 g/day of L-methionine, preferably 3-8 g/day of L-methionine, and more preferably 5 g/day of L-methionine;
(vii) the dosage comprises between 1-20 g/day of betaine, preferably 8-15 g/day of betaine, and more preferably 10 g/day of betaine;
(viii) the dosage comprises 1-15 mg/day of folate, preferably 3-10 mg/day of folate, and more preferably 5 mg/day of folate; or
(ix) the dosage comprises a combination of 5 g/day of L-methionine, 10 g/day of betaine and 5 mg/day of folate.

6. The formulation for use according to claim 1 or the use of claim 2 wherein the formulation is for administration for at least 4 weeks, and preferably for at least 6 weeks.

7. The formulation for use according to claim 1, or the use of claim 2, wherein the treating further comprises administration of lorazepam to the patient.

8. The formulation for use or use of claim 7, wherein the treating comprises administration of lorazepam to the patient in a dosage of up to 2 mg/day.

9. The formulation for use according to claim 1 or the use of claim 2 wherein unipolar depression comprises a typical depression subtype symptom selected from the group consisting of sleeping less and eating less compared to a normal control index.

10. The formulation for use according to claim 1 or the use of claim 2 wherein unipolar depression comprises an atypical depression subtype symptom selected from the group consisting of sleeping more and eating more compared to a normal control index.

11. A composition for use in the treatment of a unipolar depression wherein said composition comprises a combination of L-methionine, betaine, and folate or folic acid, wherein the combination is formulated as a bar, a liquid, a cookie, and a powder and wherein folate is the anion form of folic acid.

## Patentansprüche

1. Therapeutische Formulierung aufweisend Betain, L-Methionin und Folat oder Folsäure in einer Menge, die die endogene Synthese von S-Adenosylmethionin (SAM), das in dem Ein-Kohlenstoff-Zyklus produziert wird, steigert, zur Verwendung bei der Behandlung von unipolarer Depression, wobei Folat die anionische Form von Folsäure ist.

2. Verwendung von Betain, L-Methionin und Folat oder Folsäure zur Herstellung eines Arzneimittels zur Behandlung von unipolarer Depression bei einem Patienten, der die Behandlung braucht, wobei die Behandlung eine Verabreichung einer therapeutischen Formulierung, die Betain und Folat in einer Menge aufweist, die die endogene Synthese von SAM, das in dem Ein-Kohlenstoff-Zyklus produziert wird, steigert, an den Patienten aufweist, wobei Folat die anionische Form von Folsäure ist.

3. Formulierung zur Verwendung nach Anspruch 1, oder Verwendung nach Anspruch 2, wobei ein Ansteigen der Homocystein-Spiegel bei dem Patienten 10 µmol pro Liter, im Vergleich zu einem Spiegel vor der Behandlung, nicht überschreitet.

4. Formulierung zur Verwendung nach Anspruch 1, oder Verwendung nach Anspruch 2, wobei die Menge an produziertem SAM zwischen dem 100-180%-Fachen der normalen Menge an SAM, die bei dem Patienten vor der Verabreichung produziert wurde, bevorzugt das 140%-Fache der normalen Menge an SAM, die bei dem Patienten vor der Verabreichung produziert wurde, ist.

5. Formulierung zur Verwendung nach Anspruch 1, oder Verwendung nach Anspruch 2, wobei:
(i) die Dosierung zwischen dem 1-6-Fachen des Tagesbedarfs an L-Methionin des Patienten aufweist;
(ii) die Dosierung das 1,5-4-Fache des Tagesbedarfs an L-Methionin des Patienten aufweist;
(iii) die Dosierung das 2,5-Fache des Tagesbedarfs an L-Methionin des Patienten aufweist;
(iv) die Dosierung zwischen dem 1-20-Fachen der durchschnittlichen Tagesaufnahme an Betain des Patienten, bevorzugt zwischen dem 2,5-15-Fachen der durchschnittlichen Tagesaufnahme an Betain des Patienten, und bevorzugter zwischen dem 5-10-Fachen der durchschnittlichen Tagesaufnahme an Betain des Patienten aufweist;
(v) die Dosierung zwischen dem 1-20-Fachen des Tagesbedarfs an Folat des Patienten, bevorzugt das 10-18-Fache des Tagesbedarfs an Folat des Patienten, und bevorzugter das 13-Fache des Tagesbedarfs an Folat des Patienten aufweist;
(vi) die Dosierung zwischen 1-10 g/Tag an L-Methionin, bevorzugt 3-8 g/Tag an L-Methionin, und bevorzugter 5 g/Tag an L-Methionin aufweist;
(vii) die Dosierung zwischen 1-20 g/Tag an Betain, bevorzugt 8-15 g/Tag an Betain, und bevorzugter 10 g/Tag an Betain aufweist;
(viii) die Dosierung 1-15 mg/Tag an Folat, bevorzugt 3-10 mg/Tag an Folat, und bevorzugter 5 mg/Tag an Folat aufweist; oder
(ix) die Dosierung eine Kombination von 5 g/Tag an L-Methionin, 10 g/Tag an Betain und 5 mg/Tag an Folat aufweist.

6. Formulierung zur Verwendung nach Anspruch 1, oder Verwendung nach Anspruch 2, wobei die Formulierung für eine mindestens 4 Wochen lange, und bevorzugt für eine mindestens 6 Wochen lange Verabreichung ist.

7. Formulierung zur Verwendung nach Anspruch 1, oder Verwendung nach Anspruch 2, wobei die Behandlung außerdem die Verabreichung von Lorazepam an den Patienten aufweist.

8. Formulierung zur Verwendung nach Anspruch 7, oder Verwendung nach Anspruch 7, wobei die Behandlung die Verabreichung von Lorazepam an den Patienten in einer Dosierung von bis zu 2 mg/Tag aufweist.

9. Formulierung zur Verwendung nach Anspruch 1, oder Verwendung nach Anspruch 2, wobei die unipolare Depression ein typisches Depressions-Subtypsymptom aufweist, das ausgewählt ist aus der Gruppe, die besteht aus weniger schlafen und weniger essen im Vergleich zu einem üblichen Kontroll-Index.

10. Formulierung zur Verwendung nach Anspruch 1, oder Verwendung nach Anspruch 2, wobei die unipolare Depression ein atypisches Depressions-Subtypsymptom aufweist, das ausgewählt ist aus der Gruppe, die besteht aus mehr schlafen und mehr essen im Vergleich zu einem üblichen Kontroll-Index.

11. Zusammensetzung zur Verwendung bei der Behandlung einer unipolaren Depression, wobei die Zusammensetzung eine Kombination von L-Methionin, Betain und Folat oder Folsäure aufweist, wobei die Kombination formuliert ist als ein Riegel, eine Flüssigkeit, ein Keks, und ein Pulver, und wobei Folat die anionische Form von Folsäure ist.

## Revendications

1. Formulation thérapeutique comprenant de la bétaïne, de la L-méthionine et du folate ou de l'acide folique en une quantité qui augmente la synthèse endogène de S-adénosylméthionine (SAM) produite dans le cycle à un seul carbone, pour utilisation dans le traitement de la dépression unipolaire, dans laquelle le folate est la forme anionique de l'acide folique.

2. Utilisation de bétaïne, de L-méthionine et de folate ou d'acide folique dans la fabrication d'un médicament destiné au traitement de la dépression unipolaire chez un patient en ayant besoin, dans laquelle le traitement comprend l'administration au patient d'une formulation thérapeutique comprenant de la bétaïne et du folate en une quantité qui augmente la synthèse endogène de SAM produite dans le cycle à un seul carbone, et dans laquelle le folate est la forme anionique de l'acide folique.

3. Formulation pour utilisation selon la revendication 1 ou utilisation selon la revendication 2, dans laquelle l'augmentation des niveaux d'homocystéine chez le patient ne dépasse pas 10 micromoles par litre en comparaison avec le niveau avant traitement.

4. Formulation pour utilisation selon la revendication 1 ou utilisation selon la revendication 2, dans laquelle la quantité de SAM produite est comprise entre 100 et 180 % de la quantité normale de SAM produite chez le patient avant ladite administration, de préférence de 140 % de la quantité normale de SAM produite chez le patient avant ladite administration.

5. Formulation pour utilisation selon la revendication 1 ou utilisation selon la revendication 2, dans laquelle :
(i) la dose comprend de 1 à 6 fois les besoins quotidiens en L-méthionine du patient ;
(ii) la dose comprend de 1,5 à 4 fois les besoins quotidiens en L-méthionine du patient ;
(iii) la dose comprend 2,5 fois les besoins quotidiens en L-méthionine du patient ;
(iv) la dose comprend de 1 à 20 fois l'apport quotidien moyen de bétaïne au patient, de préférence de 2,5 à 15 fois l'apport quotidien moyen de bétaïne au patient, et plus préférablement de 5 à 10 fois l'apport quotidien moyen de bétaïne au patient ;
(v) la dose comprend de 1 à 20 fois les besoins quotidiens en folate du patient, de préférence de 10 à 18 fois les besoins quotidiens en folate du patient, et plus préférablement 13 fois les besoins quotidiens en folate du patient ;
(vi) la dose comprend de 1 à 10 g/jour de L-méthionine, de préférence de 3 à 8 g/jour de L-méthionine, et plus préférablement 5 g/jour de L-méthionine ;
(vii) la dose comprend de 1 à 20 g/jour de bétaïne, de préférence de 8 à 15 g/jour de bétaïne, et plus préférablement 10 g/jour de bétaïne ;
(viii) la dose comprend de 1 à 15 mg/jour de folate, de préférence de 3 à 10 mg/jour de folate, et plus préférablement 5 mg/jour de folate ; ou
(ix) la dose comprend une combinaison de 5 g/jour de L-méthionine, de 10 g/jour de bétaïne et de 5 mg/jour de folate.

6. Formulation pour utilisation selon la revendication 1 ou utilisation selon la revendication 2, dans laquelle la formulation est destinée à être administrée pendant au moins 4 semaines, et de préférence pendant au moins 6 semaines.

7. Formulation pour utilisation selon la revendication 1 ou utilisation selon la revendication 2, dans laquelle le traitement comprend en outre l'administration de lorazépam au patient.

8. Formulation pour utilisation ou utilisation selon la revendication 7, dans laquelle le traitement comprend l'administration de lorazépam au patient à une dose allant jusqu'à 2 mg/jour.

9. Formulation pour utilisation selon la revendication 1 ou utilisation selon la revendication 2, dans laquelle la dépression unipolaire comprend un symptôme de sous-type de dépression typique choisi dans l'ensemble constitué par une perte de sommeil et une perte d'appétit en comparaison avec un indice témoin normal.

10. Formulation pour utilisation selon la revendication 1 ou utilisation selon la revendication 2, dans laquelle la dépression unipolaire comprend un symptôme de sous-type de dépression atypique choisi dans l'ensemble constitué par une augmentation du sommeil et une augmentation de l'appétit en comparaison avec un indice témoin normal.

11. Composition pour utilisation dans le traitement d'une dépression unipolaire, laquelle composition comprend une combinaison de L-méthionine, de bétaïne et de folate ou d'acide folique, dans laquelle la combinaison est formulée sous la forme d'une barre, d'un liquide, d'un cookie ou d'une poudre, et dans laquelle le folate est la forme anionique de l'acide folique.
